# EUROPEAN PATENT APPLICATION

(11) **EP 4 837 897 A1**
(43) Date of publication of application: **11.11.2026**
(21) Application number: 24915241.4
(22) Date of filing: 31.12.2024
(51) Int. Cl.: C07C 229/16, C08G 65/00, A61K 9/127

(54) **PEG LIPID AND USE THEREOF**

(30) Priority: 02.01.2024 WO PCT/CN2024/070101; 28.06.2024 WO PCT/CN2024/102593
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN); CHEN, Yonghao, Shenzhen, Guangdong 518000 (CN)
(74) Representative: Dentons Patent Solutions Rechtsanwaltsgesellschaft mbH
(86) International application number: PCT/CN2024/144069
(87) International publication number: WO 2025/146027

(57) **Abstract**

The present disclosure relates to a PEG lipid and the use thereof. The PEG lipid can be used for preparing a lipid nanoparticle that delivers an active ingredient. The present disclosure further relates to a pharmaceutical composition and a lipid nanoparticle comprising the PEG lipid, as well as the use thereof in preparing a pharmaceutical.

## Description

### TECHNICAL FIELD

The present invention belongs to the field of biotechnology, and relates to a PEG lipid and the use thereof.

### BACKGROUND

In nucleic acid drugs RNAs or DNAs exert their effects, including mRNAs, antisense oligonucleotides (ASOs), microRNAs (miRNAs), small interfering RNAs (siRNAs), and aptamers, among others. Research has found that nucleic acid drugs have great therapeutic potential in areas such as cancer treatment, infectious disease prevention, and genetic disease treatment.

To avoid degradation by nucleases in the blood, nucleic acid drugs require delivery vehicles to deliver them into the body to exert their pharmacological effects. Common delivery vehicles for nucleic acid drugs include lipid nanoparticles (LNPs). A lipid nanoparticle generally comprises a cationic lipid, a phospholipid, a helper lipid, and a PEG lipid. Among these, the PEG lipid can both drive self-assembly and prevent particle aggregation, making the preparation of lipid nanoparticles easier. It is also generally believed that the PEG lipid can prolong the stability and residence time of lipid nanoparticles in blood circulation. However, recent research has found that upon repeated injections of lipid nanoparticles containing PEG lipids, immune reactions occur which lead to a reduction in the *in vivo* circulation time of the lipid nanoparticles and an increase in their accumulation in the liver and spleen. This phenomenon is called as "accelerated blood clearance" (ABC) phenomenon. The accelerated blood clearance phenomenon associated with PEG lipids poses severe challenges to their development and clinical application.

### SUMMARY OF THE INVENTION

In a first aspect, the present disclosure provides a PEG lipid represented by formula (I): 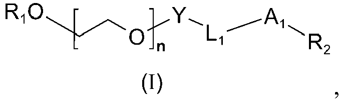 or a salt thereof, or a stereoisomer thereof,
wherein:
R₁ is H, HO-Z₁-Q₁- or H-Z₁-Q₁-,
Y is -C(=O)-, -C(=O)O-, -C(=O)NH-, -Q₂-Z₂-Q₃-, or a bond,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene or a bond,
A₁ is -C(=O)O-, -OC(=O)-, -OC(=O)O-, -O-, -S-, or a bond,
R₂ is C₄~C₂₄ hydrocarbylene,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl, and
n is any integer from 30 to 60.

In a second aspect, the present disclosure further provides a PEG lipid represented by formula (IX): 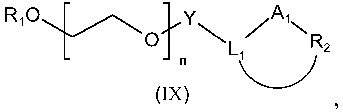 or a salt thereof, or a stereoisomer thereof,
wherein:
R₁ is H, HO-Z₁-Q₁- or H-Z₁-Q₁-,
Y is -C(=O)-, -C(=O)O-, -C(=O)NH-, -Q₂-Z₂-Q₃-, or a bond,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene, or a bond,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl, and
n is any integer from 30 to 60,
L₁ together with A₁ and R₂ forms a four-, five- or six-membered ring.

In some embodiments, L₁ in the formula (IX) is C₁~C₂₀ hydrocarbylene.

In a third aspect, the present disclosure further provides a PEG lipid represented by formula (X): 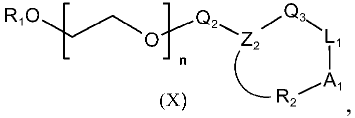 or a salt thereof, or a stereoisomer thereof,
wherein:
R₁ is H, HO-Z₁-Q₁- or H-Z₁-Q₁-,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene, or a bond,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl,
n is any integer from 30 to 60, and
Z₂ together with Q₃, L₁, A₁ and R₂ forms a four-, five- or six-membered ring.

In some embodiments, in the formula (X), Z₁ is -L₂-CH(OR₃)-L₃-, -L₂-C(=O)-L₃-, -L₂-CH(R₃)-L₃-, -L₂-, or a bond, and Z₂ is -L₂-CH(OR₃)-L₃-, -L₂-C(=O)-L₃-, -L₂-CH(R₃)-L₃- or - L₂-.

In some embodiments, R₁ is H, 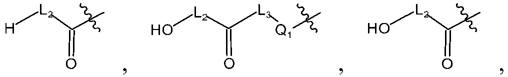 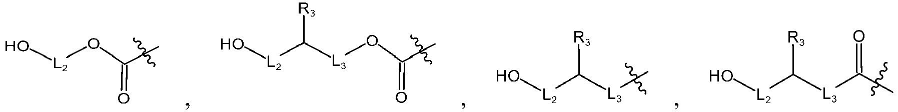 or

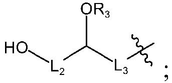

In some embodiments, R₁ is H,

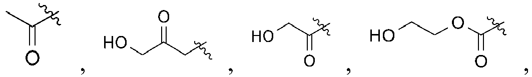

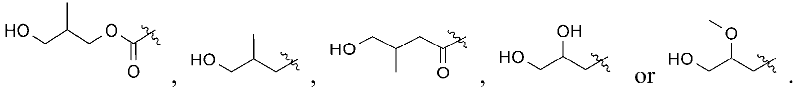

In some embodiments, the PEG lipid has a structure represented by formula (II): 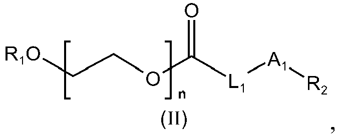 wherein R₁ is not H and is not an unsubstituted methyl.

In some embodiments, R₁ is HO-Z₁-Q₁-, A₁ is a bond, and Z₁ and Q₁ are not simultaneously a bond; preferably, Z₁ is -L₂-CH(OR₃)-L₃-, -L₂-C(=O)-L₃-, -L₂-CH(R₃)-L₃- or -L₂-. In some such embodiments, Z₁ is -L₂-CH(OR₃)-L₃-, -L₂-C(=O)-L₃-, -L₂-CH(R₃)-L₃- or -L₂-, and Q₁ is - C(=O)-, -OC(=O)-, or a bond. In preferred embodiments, L₂ is C₁~C₃ hydrocarbylene, L₃ is C₁~C₃ hydrocarbylene, and R₃ is H or C₁~C₃ alkyl. In more preferred embodiments, R₁ is

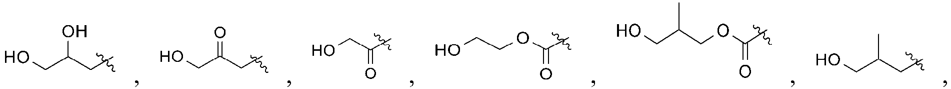

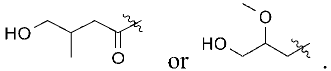

In some embodiments, R₁ is H-Z₁-Q₁-, wherein when Q₁ is a bond, Z₁ is not a bond and is not an unsubstituted methylene. In preferred embodiments, R₁ is H-Z₁-Q₁-, Z₁ is -L₂-, Q₁ is - C(=O)-, and A₁ is a bond. In such embodiments, L₂ is C₁~C₃ hydrocarbylene. In preferred embodiments, R₁ is

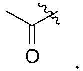

In some embodiments, the PEG lipid is represented by formula (II-1): 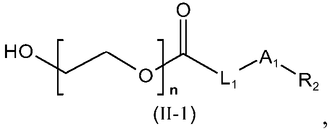 wherein A₁ is -OC(=O)-, -OC(=O)O-, -O- or -S-.

In some embodiments, the PEG lipid is represented by formula (II-1-1):

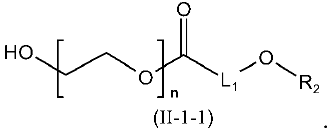

In some embodiments, the PEG lipid has a structure represented by formula (III), formula (IV), formula (V), formula (VI) or formula (VII):

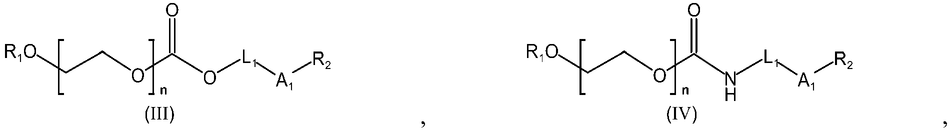

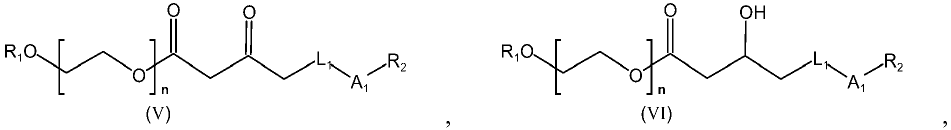

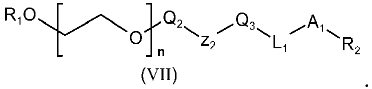

In some embodiments, the PEG lipid has the following structure:

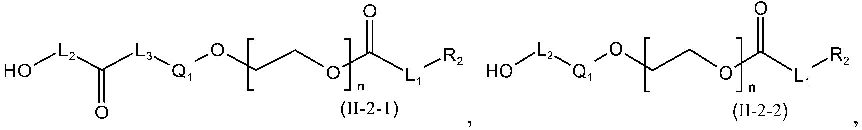

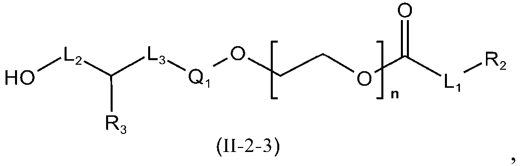

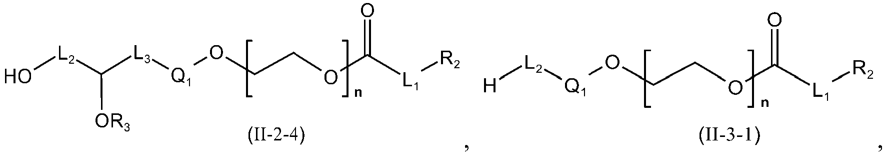

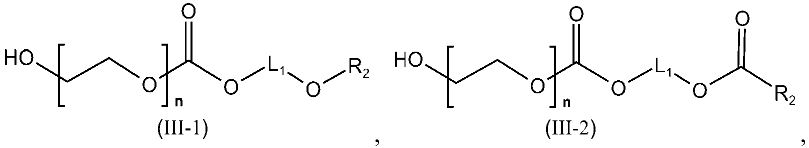

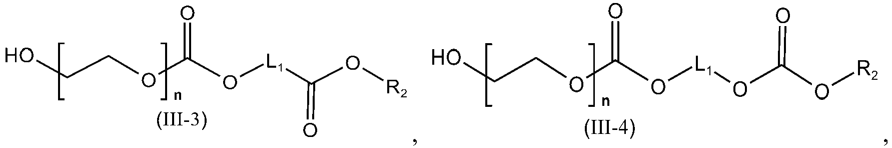

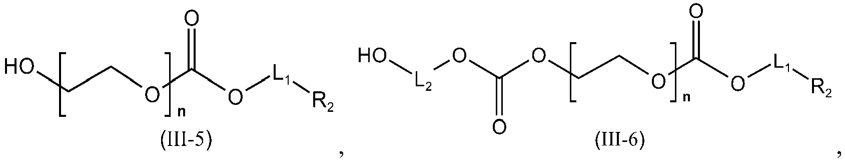

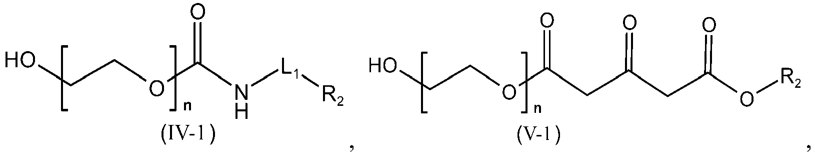

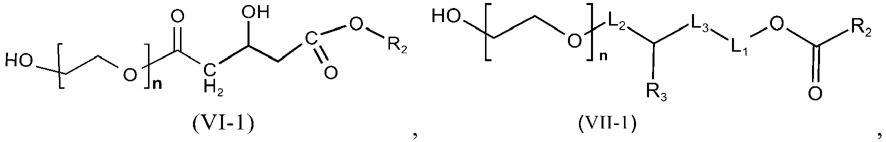

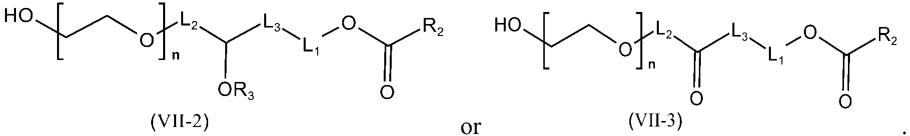

In some embodiments, the total number of carbon atoms in L₁ and R₂ is 12~25.

In some embodiments, the total number of carbon atoms in L₁ and R₂ is 16~22.

In some embodiments, L₁ is C₁~C₈ hydrocarbylene, or a bond.

In some embodiments, L₁ is C₁~C₈ alkylene, C₂~C₈ alkenylene or C₂~C₈ alkynylene.

In some embodiments, R₂ is C₇~C₂₂ hydrocarbyl.

In some embodiments, R₂ is C₇~C₂₂ alkyl, C₇~C₂₂ alkenyl or C₇~C₂₂ alkynyl.

In some embodiments, L₂ is C₁~C₃ hydrocarbylene.

In some embodiments, L₂ is C₁~C₃ alkylene, C₂~C₃ alkenylene or C₂~C₃ alkynylene.

In some embodiments, L₃ is C₁~C₂ hydrocarbylene.

In some embodiments, L₃ is C₁~C₂ alkylene, C₂ alkenylene or C₂ alkynylene.

In some embodiments, R₃ is H or C₁~C₃ alkyl.

In some embodiments, n is any integer from 30 to 55.

In some embodiments, n is any integer from 35 to 50.

In some embodiments, n is any integer from 40 to 50.

In some embodiments, the PEG lipid is: 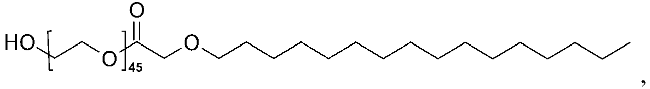 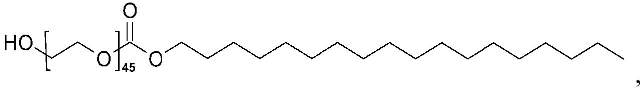 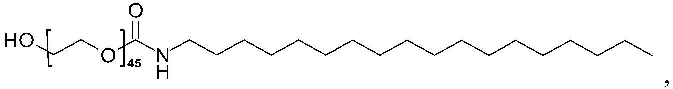 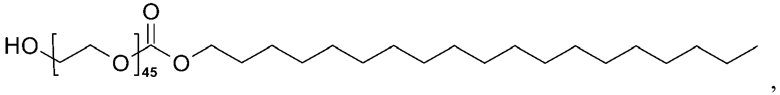 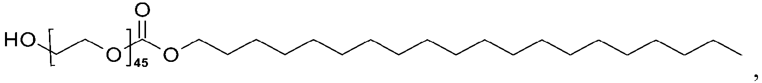 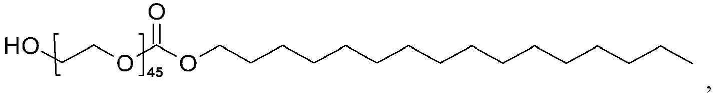 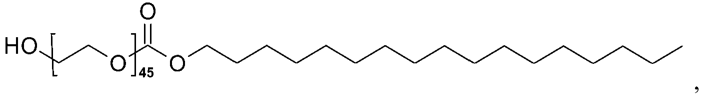 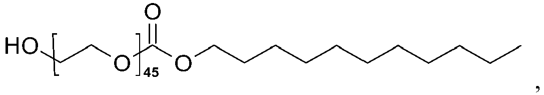 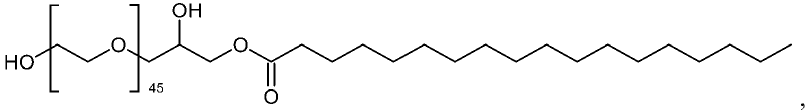 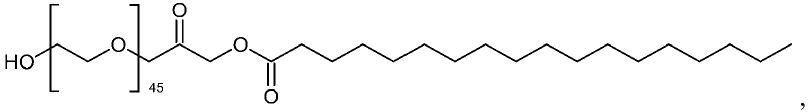 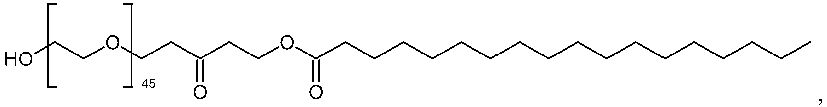 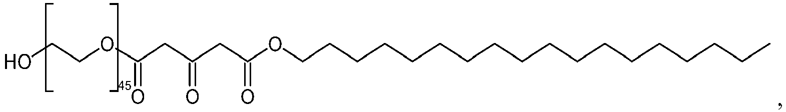 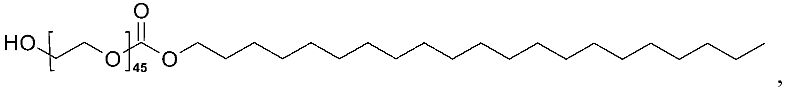 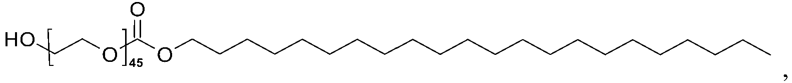 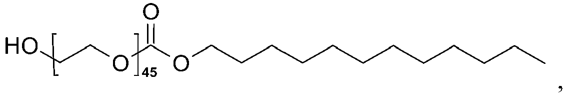 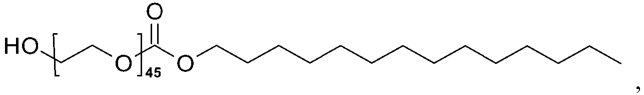 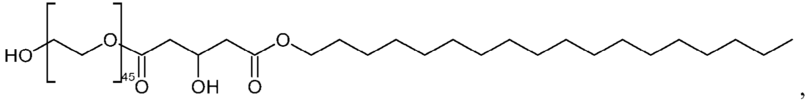 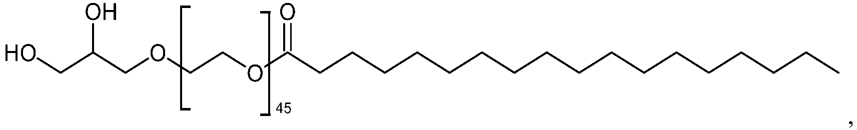 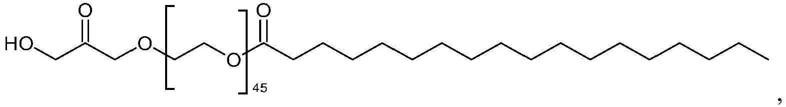 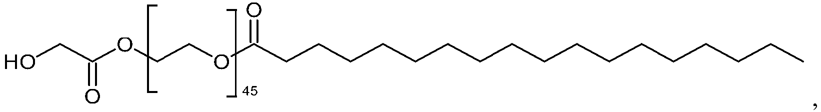 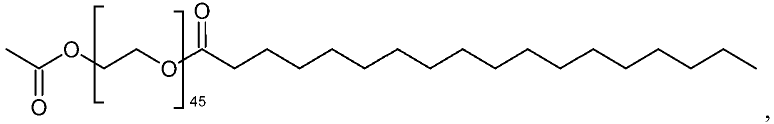 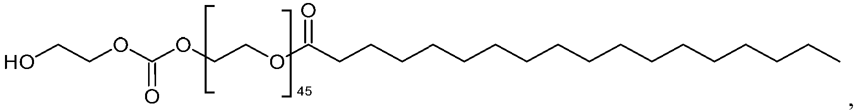 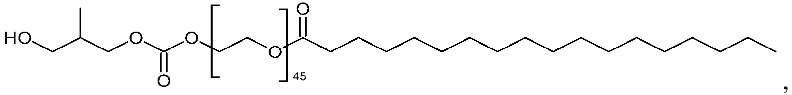 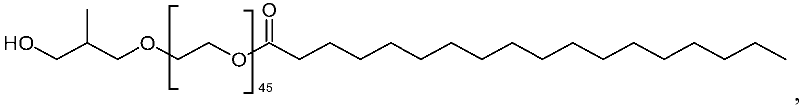 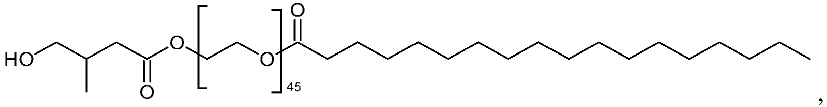 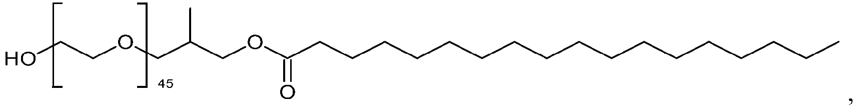 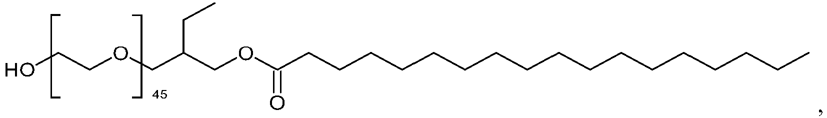 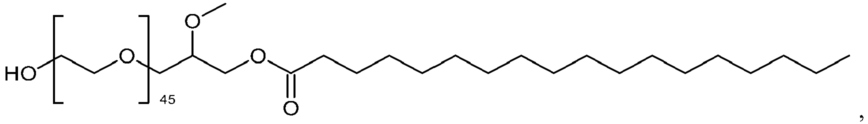 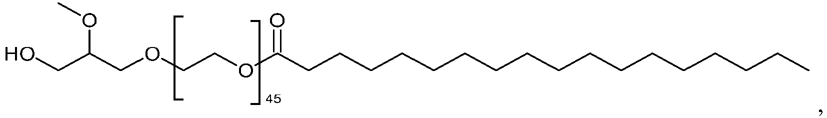 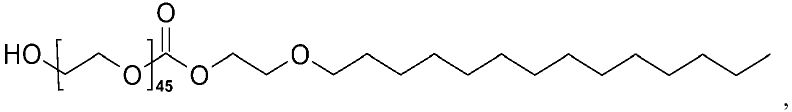 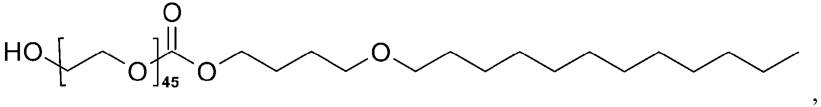 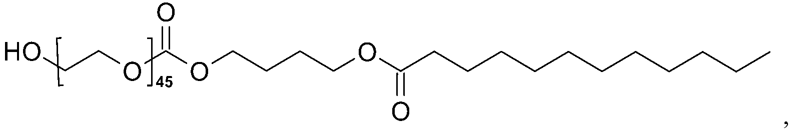 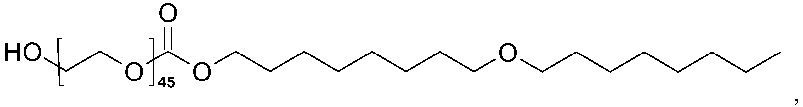 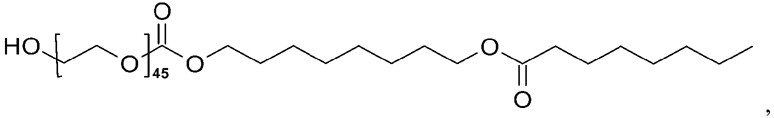 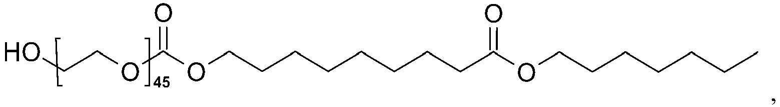 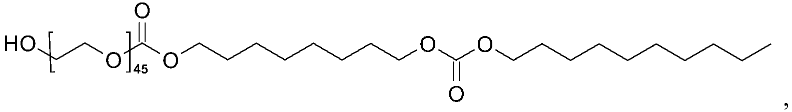 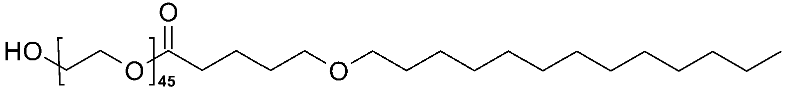 or

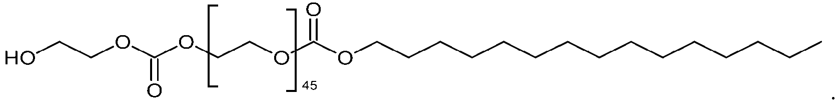

In another aspect, the present disclosure provides a use of the PEG lipid according to any of the preceding embodiments in the preparation of a lipid nanoparticle.

In another aspect, the present disclosure provides a lipid nanoparticle comprising the PEG lipid according to any of the preceding embodiments.

In some embodiments, the lipid nanoparticle further comprises an active ingredient. In some embodiments, the active ingredient is a pharmaceutically active ingredient. In some embodiments, the active ingredient is a nucleic acid. In some embodiments, the pharmaceutically active ingredient is a nucleic acid.

In some embodiments, the lipid nanoparticle further comprises one or more of a cationic lipid, a helper lipid, a structural lipid, and a polymeric lipid.

In another aspect, the present disclosure provides a composition comprising the lipid nanoparticle according to any of the preceding embodiments, and a biologically acceptable carrier.

In another aspect, the present disclosure provides a pharmaceutical composition comprising the lipid nanoparticle according to any of the preceding embodiments, and a pharmaceutically acceptable carrier.

In another aspect, the present disclosure provides a use of the lipid nanoparticle according to any of the preceding embodiments or the pharmaceutical composition according to any of the preceding embodiments in the manufacture of a medicament.

In some embodiments, the medicament is useful in gene therapy, genetic vaccination, protein replacement therapy, antisense therapy, or therapy via interfering RNA.

In some embodiments, the lipid particle as described above is used as a vehicle for transferring or delivering an active ingredient. In some embodiments, the active ingredient is a pharmaceutically active ingredient. In some embodiments, the pharmaceutically active ingredient as described above is a nucleic acid.

In some embodiments, the medicament is useful in the treatment and/or prevention of a disease.

In some embodiments, the medicament is useful in the treatment and/or prevention of one or more of rare diseases, cancers, infectious diseases, autoimmune diseases, metabolic diseases, neurological diseases, cardiovascular diseases, transplant rejection, inflammatory reactions, genetic diseases, and musculoskeletal diseases.

In some embodiments, the rare diseases include one or more of osteogenesis imperfecta, Wilson's disease, spinal muscular atrophy, Huntington's disease, Rett syndrome, amyotrophic lateral sclerosis, Duchenne type muscular dystrophy, Friedrich's ataxia, methylmalonic acidemia, cystic fibrosis, glycogen storage disease 1a, glycogen storage disease III, Crigler-Najjar syndrome, ornithine transcarbamylase deficiency, propionic acidemia, phenylketonuria, hemophilia A, hemophilia B, β-thalassemia, Lafora disease, Dravet syndrome, Alexander disease, Leber's congenital amaurosis, myelodysplastic syndrome, and homocystinuria due to CBS deficiency.

In some embodiments, the cancers include one or more of hematological malignancies, lung cancer, liver cancer, kidney cancer, head and neck cancer, esophageal cancer, gastric cancer, colorectal cancer, pancreatic cancer, brain cancer, prostate cancer, gallbladder cancer, ovarian cancer, breast cancer, cervical cancer, endometrial cancer, bladder cancer, and melanoma.

In some embodiments, the infectious diseases include diseases caused by infection with one or more of viruses, fungi, and bacteria.

In some embodiments, the autoimmune diseases include one or more of acute idiopathic thrombocytopenic purpura, chronic idiopathic thrombocytopenic purpura, systemic lupus erythematosus, rheumatoid arthritis, psoriasis, inflammatory bowel disease, multiple sclerosis, celiac disease, type 1 diabetes, and diffuse toxic goiter.

In some embodiments, the genetic diseases include one or more of hemophilia, thalassemia, and Gaucher's disease.

In some embodiments, the neurological diseases include one or more of amyotrophic lateral sclerosis, Alzheimer's disease, and glioma.

In some embodiments, the medicament is a vaccine.

In some embodiments, the medicament is a nucleic acid drug, wherein the nucleic acid includes at least one of RNA, messenger RNA (mRNA), antisense oligonucleotide, DNA, plasmid, ribosomal RNA (rRNA), microRNA (miRNA), transfer RNA (tRNA), small interfering RNA (siRNA), small nuclear RNA (snRNA), small hairpin RNA (shRNA), single-guide RNA (sgRNA), and Cas9 mRNA.

In yet another aspect, the present disclosure provides a use of the lipid nanoparticle as described above in the preparation of a vehicle for transferring or delivering an active ingredient. In some embodiments, the active ingredient is a pharmaceutically active ingredient, for example a nucleic acid. In other embodiments, the active ingredient is a transfection reagent or a detection reagent.

In some embodiments, the nucleic acid includes at least one of RNA, messenger RNA (mRNA), antisense oligonucleotide, DNA, plasmid, ribosomal RNA (rRNA), microRNA (miRNA), transfer RNA (tRNA), small interfering RNA (siRNA), small nuclear RNA (snRNA), small hairpin RNA (shRNA), single-guide RNA (sgRNA), and Cas9 mRNA.

### BRIEF DESCRIPTION OF THE DRAWINGS

Figure 1 shows assay results of hEPO concentrations in the serum from rats after multiple administrations. "001" in Figure 1 represents lipid nanoparticles comprising the PEG lipid numbered 001 in Table 1, and so on.
Figure 2 shows assay results of anti-PEG IgG antibodies in the serum from rats after multiple administrations. "001" in Figure 2 represents lipid nanoparticles comprising the PEG lipid numbered 001 in Table 1, and so on.
Figure 3 shows assay results of the cytokine IL-6 in the serum from rats after administration. "001" in Figure 3 represents lipid nanoparticles comprising the PEG lipid numbered 001 in Table 1, and so on.
Figure 4 shows assay results of the cytokine IFN-γ in the serum from rats after administration. "001" in Figure 4 represents lipid nanoparticles comprising the PEG lipid numbered 001 in Table 1, and so on.

### DETAILED DESCRIPTION OF THE INVENTION

### I. DEFINITIONS

All patents, patent applications, scientific publications, manufacturer's instructions and guidelines, etc., cited herein, whether above or below, are incorporated by reference in their entirety. Nothing herein should be construed as an admission that the present disclosure is not entitled to antedate such disclosure.

Unless otherwise stated, scientific and technical terms used herein have meanings commonly understood by one of ordinary skill in the art. Moreover, terms related to protein and nucleic acid chemistry, molecular biology, cell and tissue culture, and microbiology used herein are terms widely used in respective fields (see, for example, Molecular Cloning: A Laboratory Manual, 2nd Edition, J. Sambrook et al. eds., Cold Spring Harbor Laboratory Press, Cold Spring Harbor 1989). Meanwhile, to better understand the present invention, definitions and explanations of related terms are provided below.

As used herein, the expressions "including", "comprising", "containing", and "having" are open-ended and mean including the listed elements, steps or components, but not excluding other unlisted elements, steps or components. The expression "consisting of" excludes any unspecified element, step, or component. The expression "consisting essentially of" means that the scope is limited to the specified elements, steps or components, plus optional elements, steps or components that do not materially affect the basic and novel properties of the claimed subject matter. It should be understood that the expressions "consisting essentially of" and "consisting of" are encompassed within the meaning of the expression "comprising".

As used herein, unless the context clearly indicates otherwise, an reference expressed in singular form with "a", "an" and "the", as well as a similar reference used in the context of describing the present invention (particularly in the context of the claims) should be construed to cover both singular and plural references. The term "one or more" or "at least one" encompasses 1, 2, 3, 4, 5, 6, 7, 8, 9, or more. The term "at least one" or "one or more" encompasses 1, 2, 3, 4, 5, 6, 7, 8, 9, or more.

Numerical ranges described herein should be understood to encompass any and all subranges contained therein. For example, the range "1 to 10" should be understood to include not only the explicitly recited values of 1 and 10, but also any individual value within the range of 1 to 10 (e.g., 2, 3, 4, 5, 6, 7, 8, and 9) and subranges (e.g., 1 to 2, 1.5 to 2.5, 1 to 3, 1.5 to 3.5, 2.5 to 4, 3 to 4.5, etc.). As another example, "C₁~C₆ alkyl" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyls. This principle also applies to ranges expressed using only one number as a minimum or maximum value.

As used herein, the terms "and/or", "any combination thereof" and their grammatical equivalents are used interchangeably. These terms can denote that any combination is specifically intended. For example, the phrases "A, B and/or C" or "A, B, C, or any combination thereof" can refer to "A alone; B alone; C alone; A and B; B and C; A and C; and A, B, and C".

Unless otherwise stated, all methods described herein can be performed in any suitable order.

As used herein, the term "hydrocarbyl" refers to the group remaining after the loss of one hydrogen atom from an aliphatic hydrocarbon, including straight or branched, saturated or unsaturated hydrocarbyl groups. A hydrocarbyl group includes, but is not limited to, alkyl, alkenyl, and alkynyl groups. In some embodiments, the hydrocarbyl group has from 1 to 24 carbon atoms (C₁~C₂₄ hydrocarbyl), for example, 1, 2, 3, 4, 5, 6, 7, 8, 9, 10, 11, 12, 13, 14, 15, 16, 17, 18, 19, 20, 21, 22, 23 or 24 carbon atoms (C₁, C₂, C₃, ... C₂₁, C₂₂, C₂₃, or C₂₄ hydrocarbyl). Examples of the hydrocarbyl group include, but are not limited to, C₁~C₂₄ hydrocarbyl, C₁~C₂₂ hydrocarbyl, C₁~C₂₀ hydrocarbyl, C₁~C₁₈ hydrocarbyl, C₁~C₁₆ hydrocarbyl, C₁~C₁₂ hydrocarbyl, C₁~C₁₀ hydrocarbyl, C₁~C₈ hydrocarbyl, C₁~C₇ hydrocarbyl, C₁~C₆ hydrocarbyl, C₁~C₄ hydrocarbyl, C₁~C₃ hydrocarbyl, C₁~C₂ hydrocarbyl, C₂~C₈ hydrocarbyl, C₂~C₄ hydrocarbyl, C₄~C₈ hydrocarbyl, C₄~C₉ hydrocarbyl, C₅~C₈ hydrocarbyl, C₃ hydrocarbyl, C₄ hydrocarbyl, C₅ hydrocarbyl, C₆ hydrocarbyl, C₇ hydrocarbyl, C₈ hydrocarbyl, C₉ hydrocarbyl, C₁₀ hydrocarbyl, C₁₁ hydrocarbyl, C₁₂ hydrocarbyl, C₁₃ hydrocarbyl, C₁₄ hydrocarbyl, C₁₅ hydrocarbyl, C₁₆ hydrocarbyl, C₁₇ hydrocarbyl, C₁₈ hydrocarbyl, C₁₉ hydrocarbyl, C₂₀ hydrocarbyl, C₂₁ hydrocarbyl and C₂₂ hydrocarbyl. Unless explicitly stated otherwise in this specification, the hydrocarbyl group is optionally substituted, and for the substituents, reference is made to the definition of "optionally substituted" below. In certain embodiments, the hydrocarbyl group has no branches (i.e., is a straight chain), one branch, two branches, or multiple branches.

As used herein, the term "hydrocarbylene" refers to a divalent group remaining after further loss of one hydrogen atom from the hydrocarbyl as defined above. Unless expressly stated otherwise in this specification, the hydrocarbylene group is also optionally substituted.

As used herein, the term "alkyl" is a straight or branched saturated monovalent hydrocarbyl. In some embodiments, an alkyl group has from 1 to 24 carbon atoms (C₁~C₂₄ alkyl), for example, 1, 2, 3, 4, 5, 6, 7, 8, 9, 10, 11, 12, 13, 14, 15, 16, 17, 18, 19, 20, 21, 22, 23 or 24 carbon atoms (C₁, C₂, C₃, ...C₂₁, C₂₂, C₂₃, or C₂₄ alkyl). Examples of the alkyl group include, but are not limited to, C₁~C₂₄ alkyl, C₁~C₂₂ alkyl, C₁~C₂₀ alkyl, C₁~C₁₈ alkyl, C₁~C₁₆ alkyl, C₁~C₁₂ alkyl, C₁~C₁₀ alkyl, C₁~C₈ alkyl, C₁~C₅ alkyl, C₁~C₆ alkyl, C₁~C₄ alkyl, C₁~C₃ alkyl, C₁~C₂ alkyl, C₂~C₈ alkyl, C₂~C₄ alkyl, C₄~C₈ alkyl, C₄~C₉ alkyl, C₅~C₈ alkyl, methyl, ethyl, n-propyl, isopropyl, n-butyl, sec-butyl, isobutyl, tert-butyl, n-pentyl, n-hexyl, n-heptyl, n-octyl, n-nonyl, n-decyl, n-undecyl, n-dodecyl, n-tridecyl, and tridecan-7-yl. Unless explicitly stated otherwise in this specification, the alkyl group is optionally substituted.

As used herein, the term "alkylene" refers to a divalent group remaining after further loss of one hydrogen atom from the alkyl as defined above. Unless expressly stated otherwise in this specification, the alkylene is also optionally substituted.

As used herein, the term "alkenyl" is a straight or branched monovalent hydrocarbyl containing one or more double bonds (C=C). In some embodiments, an alkenyl group has from 2 to 24 carbon atoms (C₂-C₂₄ alkenyl), for example, 2, 3, 4, 5, 6, 7, 8, 9, 10, 11, 12, 13, 14, 15, 16, 17, 18, 19, 20, 21, 22, 23 or 24 carbon atoms (C₂, C₃, C₄, ...C₂₁, C₂₂, C₂₃, or C₂₄ alkenyl), and has 1, 2, 3, 4, or more double bonds. The alkenyl group includes, but is not limited to, C₂~C₂₄ alkenyl, C₂~C₂₂ alkenyl, C₂~C₂₀ alkenyl, C₂~C₁₈ alkenyl, C₂~C₁₆ alkenyl, C₂~C₁₂ alkenyl, C₂~C₁₀ alkenyl, C₂~C₈ alkenyl, C₂~C₇ alkenyl, C₂~C₆ alkenyl, C₂~C₄ alkenyl, C₂~C₃ alkenyl, C₄~C₈ alkenyl, C₄~C₉ alkenyl, C₅~C₈ alkenyl having 1, 2, 3, 4 or more double bonds. Some more specific examples include, but are not limited to, ethenyl, propenyl, but-1-enyl, but-2-enyl, pent-1-enyl, pent-2-enyl, hex-1-enyl, hex-2-enyl, hex-3-enyl, hept-1-enyl, hept-2-enyl, hept-3-enyl, oct-1-enyl, oct-2-enyl, oct-3-enyl, non-1-enyl, non-2-enyl, and non-3-enyl. In some embodiments, the alkenyl group has one double bond. Unless expressly stated otherwise in this specification, the alkenyl group is optionally substituted.

As used herein, the term "alkenylene" refers to a divalent group remaining after further loss of one hydrogen atom from the alkenyl as defined above. Unless expressly stated otherwise in the specification, the alkenylene group is also optionally substituted.

As used herein, the term "alkynyl" is a straight or branched monovalent hydrocarbyl group containing one or more triple bonds (C≡C). In some embodiments, an alkynyl group has from 2 to 24 carbon atoms (C₂~C₂₄ alkynyl), for example, 2, 3, 4, 5, 6, 7, 8, 9, 10, 11, 12, 13, 14, 15, 16, 17, 18, 19, 20, 21, 22, 23 or 24 carbon atoms (C₂, C₃, C₄, ... C₂₁, C₂₂, C₂₃, or C₂₄ alkynyl), and having 1, 2, 3, 4, or more triple bonds. The alkynyl group includes, but is not limited to, C₂~C₂₄ alkynyl, C₂~C₂₂ alkynyl, C₂~C₂₀ alkynyl, C₂~C₁₈ alkynyl, C₂~C₁₆ alkynyl, C₂~C₁₂ alkynyl, C₂~C₁₀ alkynyl, C₂~C₈ alkynyl, C₂~C₇ alkynyl, C₂~C₆ alkynyl, C₂~C₄ alkynyl, C₂~C₃ alkynyl, C₄~C₈ alkynyl, C₄~C₉ alkynyl, C₅~C₈ alkynyl having 1, 2, 3, 4 or more triple bonds. Some more specific examples include, but are not limited to, ethynyl, propynyl, but-1-ynyl, but-2-ynyl, pent-1-ynyl, pent-2-ynyl, hex-1-ynyl, hex-2-ynyl, hex-3-ynyl, hept-1-ynyl, hept-2-ynyl, hept-3-ynyl, oct-1-ynyl, oct-2-ynyl, oct-3-ynyl, non-1-ynyl, non-2-ynyl, and non-3-ynyl. In some embodiments, the alkynyl group has one triple bond. Unless explicitly stated otherwise in this specification, the alkynyl group is optionally substituted.

As used herein, the term "alkynylene" refers to a divalent group remaining after further loss of one hydrogen atom from the alkynyl as defined above. Unless explicitly stated otherwise in the specification, the alkynylene group is also optionally substituted.

As used herein, the term "optionally substituted" means that one or more hydrogen atoms attached to an atom or group are independently unsubstituted, or independently substituted with one or more (e.g., 1, 2, 3, or 4) substituents. The substituents may be independently selected from, but are not limit to, a halogen (such as a chlorine, bromine, fluorine, or iodine), a carboxylic acid (such as -C(=O)OH), an oxygen (such as =O), a sulfur (such as =S), a hydroxyl (such as -OH), an ester group (such as -C(=O)ORiii or -OC(=O)Riii), an aldehyde group (such as -C(=O)H), a carbonyl (such as -C(=O)Riii, or represented by C=O), an acyl halide (such as -C(=O)X, wherein X is selected from bromine, fluorine, chlorine, or iodine), a carbonic ester group (such as -OC(=O)ORiii), an alkoxy (such as -ORiii), an acetal (such as - C(ORiii)₂Riii, wherein each ORiii is the same or different and is an alkoxy group), a phosphate (such as P(=O)₄³⁻), a thiol (such as -SH), a sulfoxide (such as -S(=O)Riii), a sulfinic acid (such as -S(=O)OH), a sulfonic acid (such as -S(=O)₂OH), a thioaldehyde (such as -C(=S)H), a sulfate (such as S(=O)₄²⁻), a sulfonyl (such as -S(=O)₂Riii), a sulfinyl (such as -S(=O)Riii), an amide group (such as -C(=O)N(Riii)₂ or -N(Riii)C(=O)Riii), an azido (such as -N₃), a nitro (such as - NO₂), a cyano (such as -CN), an isocyano (such as -NC), an acyloxy (such as -OC(=O)Riii), an amino (such as -N(Riii)₂, -N(Riii)H or -NH₂), a carbamoyl (such as -OC(=O)N(Riii)₂, - OC(=O)N(Riii)H or -OC(=O)NH₂), a sulfonamide group (such as -S(=O)₂N(Riii)₂, - S(=O)₂N(Riii)H, -S(=O)₂NH₂, -N(Riii)S(=O)₂Riii, -N(H)S(=O)₂Riii, -N(Riii)S(=O)₂H or - N(H)S(=O)₂H), an alkyl, an alkenyl, an alkynyl, a cyclohydrocarbyl (such as cycloalkyl, cycloalkenyl or cycloalkynyl), a heterocyclohydrocarbyl (such as heterocycloalkyl containing one or more heteroatoms selected from S, N, and O, or heterocycloalkenyl containing one or more heteroatoms selected from S, N, and O), an aryl (such as phenyl, or a fused ring group), a heteroaryl (such as an 8- to 10-membered bicyclic heteroaryl containing 1 to 4 heteroatoms independently selected from nitrogen, oxygen, or sulfur), -C(=O)SRiii, -C(=N-CN)N(Riii)₂, - C(=N-O-CH₃)N(Riii)₂, -C(=N-SO₂-NH₂)N(Riii)₂, -C(=CH-NO₂)N(Riii)₂, -OC(=O)N(Riii)₂, - CHN(Riii)N(Riii)₂, -C(=O)N(Riii)ORiii, -N(Riii)₂C(=O)ORiii, -OP(=O)(ORiii)₂, - P(=O)(ORiii)₂, -N(ORiii)C(=O)Riii, -N(ORiii)S(=O)₂Riii, -N(ORiii)C(=O)ORiii, - N(ORiii)C(=O)N(Riii)₂, -N(ORiii)C(=S)N(Riii)₂, -N(ORiii)C(NRiii)N(Riii)₂, - N(ORiii)C(CHRiii)N(Riii)₂. In any of the foregoing, Riii is a hydrogen, or alkyl, or alkenyl, or alkynyl, or heteroalkyl, or heteroalkenyl, or heteroalkynyl, as defined herein. In some embodiments, Riii is a hydrogen, or C₁-C₁₂ alkyl, or C₂-C₁₂ alkenyl, or C₂-C₁₂ alkynyl, or C₂-C₁₂ heteroalkyl, or C₃-C₁₂ heteroalkenyl, or C₃-C₁₂ heteroalkynyl, as defined herein.

In certain embodiments, the substituents itself may be further substituted with, for example, one or more substituents as defined herein. For example, the C₁-C₆ alkyl as a substituent may be further substituted with one or more substituents as define herein.

As use herein, that term "halo" or "halogen" group is defined to include F, Cl, Br, or I.

As used herein, "pharmaceutically acceptable salt" refers to an acid-addition salt or a base-addition salt of a compound of the present disclosure which retains the biological effectiveness and properties of the compound of the present disclosure and is not typically biologically or otherwise undesirable. In many cases, the compound of the present invention can form an acid and/or base salt due to the presence of an amino and/or carboxyl group or a similar group.

Pharmaceutically acceptable acid addition salts can be formed from the compound of the present disclosure and inorganic and/or organic acids, the inorganic acids being such as, but not limited to, hydrochloric acid, hydrobromic acid, sulfuric acid, nitric acid, carbonic acid and phosphoric acid; and the organic acids being such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphanic acid, camphor-10-sulfonic acid, capric acid, hexanoic acid, octanoic acid, cinnamic acid, citric acid, cyclamic acid, dodecyl sulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, and undecylenic acid.

Pharmaceutically acceptable base addition salts can be formed from the compound of the present disclosure and inorganic and/or organic bases. Salts derived from inorganic bases include, but are not limited to, sodium, potassium, lithium, ammonium, calcium, magnesium, iron, zinc, copper, manganese, aluminum salts, etc. For example, inorganic salts are ammonium, sodium, potassium, calcium, and magnesium salts. Salts derived from organic bases include, but are not limited to, salts of primary amines, secondary amines, tertiary amines, substituted amines (including naturally occurring substituted amines), cyclic amines, and basic ion exchange resins as following: such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucosamine, theobromine, triethanolamine, tromethamine, purine, piperazine, piperidine, N-ethylpiperidine, and polyamine resins. For example, organic bases are isopropylamine, diethylamine, ethanolamine, trimethylamine, dicyclohexylamine, choline, caffeine and the like.

As use herein, that term "isomer" means different compounds having the same molecular formula. "Stereoisomers" are isomers that differ only in the way the atoms are arranged in space. "Atropisomers" are stereoisomers resulting from hindered rotation about a single bond. "Enantiomers" are a pair of stereoisomers that are non-overlapping mirror images of each other. A mixture of any ratio of a pair of enantiomers may be referred to as a "racemic" mixture. "Diastereoisomers" are stereoisomers that have at least two asymmetric atoms and are not mirror-images of one another. "Tautomers" refer to isomeric forms of a compound that are in equilibrium with each other. The concentration of the isomeric form will depend on the environment in which the compound is found and may vary, for example, depending on whether the compound is a solid or in an organic or aqueous solution.

In certain embodiments, "stereoisomers" may also include the E and Z isomers, or mixtures thereof, as well as the *cis* and *trans* isomers, or mixtures thereof.

As used herein, the term "nucleic acid" generally refers to any compound that comprises a polymer of deoxyribonucleotides (deoxyribonucleic acid, abbreviated as DNA) or a polymer of ribonucleotides (ribonucleic acid, abbreviated as RNA) or a combination thereof. Additionally, the nucleic acid herein also includes a derivative of a nucleic acid. The term "derivative of a nucleic acid" includes chemical derivatization of a nucleic acid on a base, sugar, or phosphate of a nucleotide, as well as a nucleic acid containing an unnatural nucleotide or a nucleotide analog. Furthermore, the nucleic acid herein can be in the form of a single-stranded or double-stranded, linear or covalently closed circular molecule.

As used herein, the term "plasmid" generally refers to a circular DNA molecule, but the term can also encompass a linearized DNA molecule. Specifically, the term "plasmid" also encompasses a molecule obtained through linearizing a circular plasmid, by for example, digesting the circular plasmid with a restriction enzyme, thereby converting the circular plasmid molecule into a linear molecule. Plasmids are capable of replication, i.e., amplification independent of the genetic information stored as chromosomal DNA within a cell, and can be used for cloning, i.e., for amplifying genetic information in bacterial cells. In one optional specific example, the DNA plasmid is a medium-copy or high-copy plasmid. In another optional specific example, the DNA plasmid is a high-copy plasmid. Examples of such a high-copy plasmid includes, for example, pUC and pTZ plasmids or any other plasmid containing a replication origin that supports high-copy number of the plasmid (e.g., pMB1, pCoIE1).

As used herein, the term "vaccine" is typically understood to mean a prophylactic or therapeutic material providing at least one antigen or functioning as an antigenic, capable of stimulating the body's adaptive immune system to provide an adaptive immune response.

As used herein, the term "treatment" and the like is used herein to generally mean obtaining a desired pharmacological and/or physiological effect. Therefore, the treatment in the present application may involve the therapy of a pathological state, but may also involve the prophylactic treatment in terms of completely or partially preventing a disease or a symptom thereof. Preferably, in some embodiments, the term "treatment" should be understood as being therapeutic in terms of partially or completely curing a disease and/or adverse effects and/or symptoms attributable to the disease. Treatment can also be prophylactic or preventive treatment, i.e., measures taken to prevent a disease, e.g., to prevent an infection and/or onset of a disease.

As used herein, the term "administration" means to offer or administer an agent to a subject by any effective route. Exemplary routes of administration include, but are not limited to, one or more of injection (e.g., subcutaneous, intramuscular, intradermal, intraperitoneal, intrathecal, intraventricular, or intravenous), oral, intrabiliary, sublingual, rectal, transdermal, intranasal, vaginal, and inhalation. When used to treat a disease, disorder or condition, or a symptom thereof, administration of a substance is typically performed after onset of the disease, disorder or condition, or a symptom thereof. When used to prevent a disease, disorder or condition, or a symptom, administration of a substance is typically performed before onset of the disease, disorder or condition, or the symptom.

As used herein, the terms "subject" and "patient" are used interchangeably. In certain embodiments, the subject is a mammal such as a human, a non-human primate (e.g., ape, chimpanzee, monkey, and orangutan), a domestic animal (including dogs and cats, and livestock such as horses, cattle, pigs, sheep, and goats), or other mammals. Other mammals include, but are not limited to, mice, rats, guinea pigs, rabbits, hamsters, etc. In specific embodiments, the subject is a human. In one embodiment, the subject is a mammal (e.g., a human) suffering from an infectious disease or a neoplastic disease. In another embodiment, the subject is a mammal (e.g., a human) at risk of developing an infectious disease or a neoplastic disease.

As used herein, the term "cationic lipid" refers to a lipid that becomes positively charged when the pH is lowered below the pKa of the ionizable group of the lipid, but gradually becomes neutral at higher pH values. At a pH value below the pKa, the positively charged lipid can bind to a negatively charged nucleic acid. In certain embodiments, the cationic lipid comprises a zwitterionic lipid.

As used herein, the term "pharmaceutically acceptable" means approval by a regulatory authority (e.g., China Food and Drug Administration (CFDA), U.S. Food and Drug Administration (FDA)) or a recognized pharmacopoeia (e.g., Chinese Pharmacopoeia, European Pharmacopoeia) for use in animals and/or humans.

Herein, some elements of the present application will be described, which elements are set forth together with specific embodiments; however, it should be understood that they can be combined in any manner and in any number to produce additional embodiments. Various examples and preferred embodiments as described should not be interpreted as limiting the present application only to the explicitly described embodiments. This specification should be understood to support and include embodiments combining the explicitly described embodiments with any number of the disclosed and/or preferred elements. Furthermore, unless the context indicates otherwise, any arrangement and combination of all described elements in the present application should be considered as disclosed by the specification of the present application.

### II. PEG LIPIDS

The present disclosure provides a PEG lipid represented by formula (I): 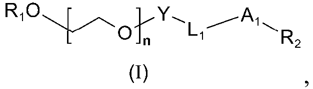 or a salt thereof, or a stereoisomer thereof,
wherein:
R₁ is H, HO-Z₁-Q₁- or H-Z₁-Q₁-,
Y is -C(=O)-, -C(=O)O-, -C(=O)NH-, -Q₂-Z₂-Q₃-, or a bond,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene, or a bond,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl, and
n is any integer from 30 to 60.

The present disclosure also provides another PEG lipid represented by formula (IX): 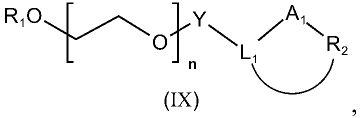 or a salt thereof, or a stereoisomer thereof,
wherein:
R₁ is H, HO-Z₁-Q₁- or H-Z₁-Q₁-,
Y is -C(=O)-, -C(=O)O-, -C(=O)NH-, -Q₂-Z₂-Q₃-, or a bond,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene, or a bond,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl,
n is any integer from 30 to 60, and
L₁ together with A₁ and R₂ forms a four-, five- or six-membered ring.

In some embodiments, L₁ in the formula (IX) is C₁~C₂₀ hydrocarbylene.

Furthermore, the present disclosure also provides a PEG lipid represented by formula (X): 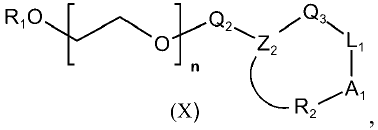 or a salt thereof, or a stereoisomer thereof,
wherein:
R₁ is H, HO-Z₁-Q₁- or H-Z₁-Q₁-,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene, or a bond,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl,
n is any integer from 30 to 60, and
Z₂ together with Q₃, L₁, A₁ and R₂ forms a four-, five- or six-membered ring.

In some embodiments, in the formula (X), Z₁ is -L₂-CH(OR₃)-L₃-, -L₂-C(=O)-L₃-, -L₂-CH(R₃)-L₃-, -L₂-, or a bond, and Z₂ is -L₂-CH(OR₃)-L₃-, -L₂-C(=O)-L₃-, -L₂-CH(R₃)-L₃- or - L₂-.

In some embodiments, the PEG lipid represented by formula (I) has a structure represented by formula (II): 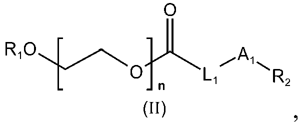 wherein A₁, L₁, R₁, R₂, and n are as defined in the formula (I), and R₁ is not H and is not an unsubstituted methyl.

In some embodiments, the PEG lipid represented by formula (I) has a structure represented by formula (III): 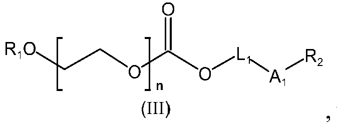 wherein A₁, L₁, R₁, R₂, and n are as defined in the formula (I).

In some embodiments, the PEG lipid represented by formula (I) has a structure represented by formula (IV): 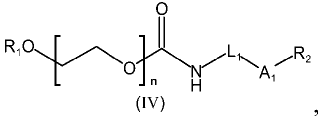 wherein A₁, L₁, R₁, R₂, and n are as defined in the formula (I).

In some embodiments, the PEG lipid represented by formula (I) has a structure represented by formula (V): 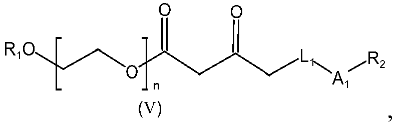 wherein A₁, L₁, R₁, R₂, and n are as defined in the formula (I).

In some embodiments, the PEG lipid represented by formula (I) has a structure represented by formula (VI): 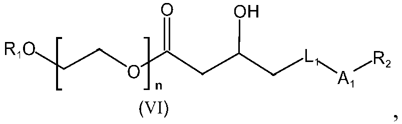 wherein A₁, L₁, R₁, R₂, and n are as defined in the formula (I).

In some embodiments, the PEG lipid shown in formula (I) has a structure represented by formula (VII): 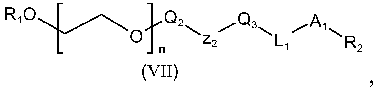 wherein A₁, L₁, R₁, R₂, Q₂, Z₂, Q₃, and n are as defined in the formula (I).

The PEG lipid represented by formula (I), formula (II), formula (III), formula (IV), formula (V), formula (VI), formula (VII), formula (VIII), formula (IX) or formula (X) includes, when applicable, one or more of the following features.

In some embodiments, the salt is a pharmaceutically acceptable salt.

In some embodiments, R₁ is H or C₁~C₉ hydrocarbyl.

In some embodiments, R₁ is H, C₁~C₉ alkyl, C₂~C₉ alkenyl or C₂~C₉ alkynyl.

In some embodiments, R₁ is not H and is not an unsubstituted methyl.

In some embodiments, R₁ is a substituted methyl or is a C₂~C₉ hydrocarbyl.

In some embodiments, R₁ is a substituted methyl or is a C₂~C₆ hydrocarbyl.

In some embodiments, R₁ is a substituted methyl, is a C₂~C₆ alkyl, is a C₂~C₆ alkenyl or is a C₂~C₆ alkynyl.

In some embodiments, R₁ is a substituted hydrocarbyl. In some embodiments, R₁ is a substituted C₁~C₉ hydrocarbyl. In some embodiments, R₁ is a substituted C₁~C₆ hydrocarbyl.

In some embodiments, R₁ is HO-Z₁-Q₁-, wherein Z₁ and Q₁ are as defined in the formula (I).

In some embodiments, R₁ is H-Z₁-Q₁-, wherein Z₁ and Q₁ are as defined in the formula (I), and when Q₁ is a bond, Z₁ is not a bond and is not an unsubstituted methylene.

In some embodiments, R₁ is HO-Z₁-Q₁-, wherein Z₁ and Q₁ are as defined in the formula (I), and Z₁ and Q₁ are not simultaneously a bond.

In some embodiments, R₁ is HO-Z₁-Q₁-, wherein Q₁ is as defined in the formula (I), and Z₁ is -L₂-CH(OR₃)-L₃-, -L₂-C(=O)-L₃-, -L₂-CH(R₃)-L₃- or -L₂-.

In some embodiments, R₁ is HO-Z₁-Q₁-, Z₁ is -L₂-CH(OR₃)-L₃-, -L₂-C(=O)-L₃-, -L₂-CH(R₃)-L₃- or -L₂-, and Q₁ is -C(=O)-, -OC(=O)-, or a bond. In some preferred embodiments, L₂ is C₁~C₃ hydrocarbylene, L₃ is C₁~C₃ hydrocarbylene, and R₃ is H or C₁~C₃ alkyl. Preferably, L₂ and L₃ are each straight C₁~C₃ alkylene, more preferably C₁ alkylene or C₂ alkylene. In more preferred embodiments, R₁ is

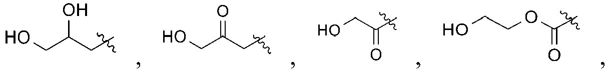

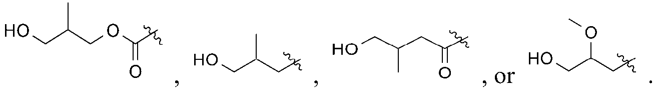

In some embodiments, R₁ is H-Z₁-Q₁-, Z₁ is -L₂-, and Q₁ is -C(=O)-. In some preferred embodiments, L₂ is C₁~C₃ hydrocarbylene. In some preferred embodiments, R₁ is

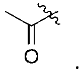

In some embodiments, R₁ is H, 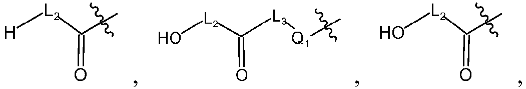 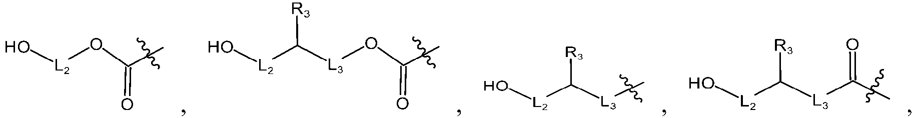 or 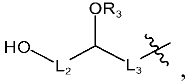 wherein Q₁, L₂, L₃, and R₃ are as defined in the formula (I).

In some embodiments, R₁ is H,

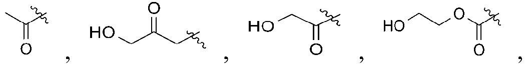

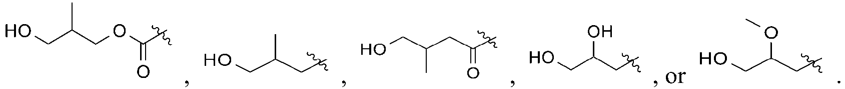

In some embodiments, R₁ is 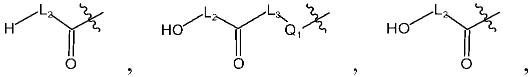 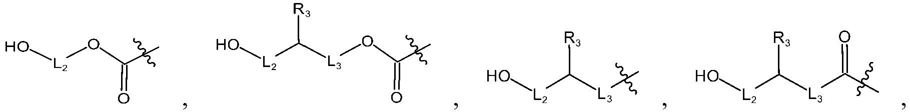 or

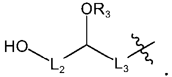

In some embodiments, R₁ is

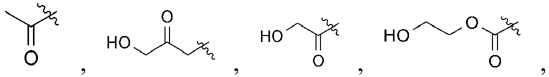

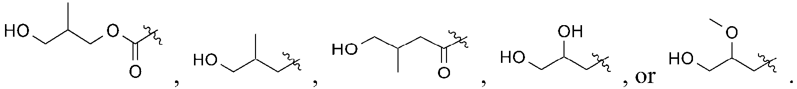

In some embodiments, R₁ is H or HO-Z₁-Q₁-. In some embodiments, R₁ is H or

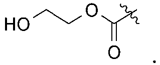

In some embodiments, R₁ is HO-Z₁-Q₁-, Z₁ is -L₂-, -L₂-CH(OR₃)-L₃-, -L₂-C(=O)-L₃- or - L₂-CH(R₃)-L₃-, and Q₁ is -OC(=O)-.

In some embodiments, R₁ is HO-Z₁-Q₁-, wherein Z₁ is -L₂-, Q₁ is -OC(=O)-, and L₂ is as defined in the formula (I).

In some embodiments, R₁ is HO-Z₁-Q₁-, wherein Z₁ is -L₂-, Q₁ is -OC(=O)-, and L₂ is C₁~C₃ hydrocarbylene.

In some embodiments, R₁ is HO-Z₁-Q₁-, wherein Z₁ is -L₂-, Q₁ is -OC(=O)-, and L₂ is C₁~C₃ alkylene or C₂~C₃ alkenylene.

In some embodiments, R₁ is H.

In some embodiments, A₁ is -C(=O)O-, -OC(=O)-, -OC(=O)O-, -O-, or a bond.

In some embodiments, A₁ is -OC(=O)-, -OC(=O)O-, -O- or -S-.

In some embodiments, A₁ is -C(=O)O-, -OC(=O)-, -O-, or a bond.

In some embodiments, A₁ is -O-, or a bond.

In some embodiments, A₁ is a bond.

In some embodiments, A₁ is -C(=O)O-.

In some embodiments, A₁ is -OC(=O)-.

In some embodiments, Q₂ is a bond or -C(=O)-.

In some embodiments, Q₂ is a bond.

In some embodiments, Q₃ is a bond.

In some embodiments, Q₂ and Q₃ are bonds.

In some embodiments, Z₁ is -L₂-CH(OR₃)-L₃-, -L₂-C(=O)-L₃-, -L₂-CH(R₃)-L₃- or -L₂-.

In some embodiments, Z₂ is -L₂-CH(OR₃)-L₃-, -L₂-C(=O)-L₃- or -L₂-CH(R₃)-L₃-.

In some embodiments, L₂ at each occurrence is independently C₁~C₃ hydrocarbylene, L₃ at each occurrence is independently C₁~C₃ hydrocarbylene, and R₃ at each occurrence is independently H or C₁~C₃ alkyl.

In some embodiments, L₂ at each occurrence is independently C₁~C₃ hydrocarbylene, preferably C₁~C₃ alkylene, more preferably C₁ alkylene or C₂ alkylene.

In some embodiments, L₃ at each occurrence is independently C₁~C₃ hydrocarbylene, preferably C₁~C₃ alkylene, more preferably C₁ alkylene or C₂ alkylene.

In some embodiments, R₃ at each occurrence is independently H or C₁~C₃ alkyl.

In some embodiments, the PEG lipid represented by formula (II) has a structure represented by formula (II-1): 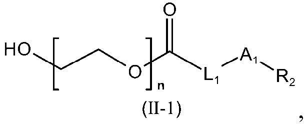 wherein A₁ is -OC(=O)-, -OC(=O)O-, -O- or -S-, and L₁, R₂, and n are as defined in the formula (II).

In some embodiments, the PEG lipid represented by formula (II) has a structure represented by 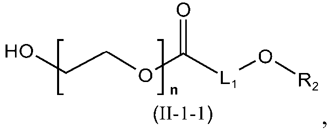 wherein L₁, R₂, and n are as defined in the formula (II).

In some embodiments, in the PEG lipid represented by formula (II), R₁ is not H and is not an unsubstituted methyl, and A₁ is -C(=O)O-, -OC(=O)-, -OC(=O)O-, -O- or -S-.

In some embodiments, in the PEG lipid represented by formula (II), R₁ is HO-Z₁-Q₁-, A₁ is -C(=O)O-, -OC(=O)-, -OC(=O)O-, -O- or -S-, wherein Z₁ and Q₁ are as defined in the formula (I), and Z₁ and Q₁ are not simultaneously a bond.

In some embodiments, in the PEG lipid represented by formula (II), R₁ is H-Z₁-Q₁-, A₁ is -C(=O)O-, -OC(=O)-, -OC(=O)O-, -O- or -S-, wherein Z₁ and Q₁ are as defined in the formula (I), and when Q₁ is a bond, Z₁ is not a bond or Z₁ is a substituted methylene.

In some embodiments, in the PEG lipid represented by formula (II), R₁ is H-Z₁-Q₁-, Q₁ is -C(=O)-, Z₁ is -L₂-, and A₁ is -C(=O)O-, -OC(=O)-, -OC(=O)O-, -O- or -S-.

In some embodiments, in the PEG lipid represented by formula (II), A₁ is a bond, and R₁ is not H and is not an unsubstituted methyl.

In some embodiments, in the PEG lipid represented by formula (II), R₁ is HO-Z₁-Q₁-, A₁ is a bond, and Z₁ and Q₁ are not simultaneously a bond. In some such embodiments, Z₁ is -L₂-CH(OR₃)-L₃-, -L₂-C(=O)-L₃-, -L₂-CH(R₃)-L₃- or -L₂-. In more preferred embodiments, Z₁ is - L₂-CH(OR₃)-L₃-, -L₂-C(=O)-L₃-, -L₂-CH(R₃)-L₃- or -L₂-, and Q₁ is -C(=O)-, -OC(=O)-, or a bond. In more preferred embodiments, L₂ is C₁~C₃ hydrocarbylene, L₃ is C₁~C₃ hydrocarbylene, and R₃ is H or C₁~C₃ alkyl. In further more preferred embodiments, L₂ and L₃ are each C₁~C₃ alkylene, more preferably straight C₁~C₃ alkylene, further more preferably C₁ alkylene or C₂ alkylene. In even more preferred embodiments, R₁ is

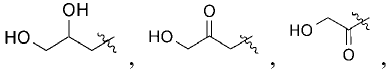

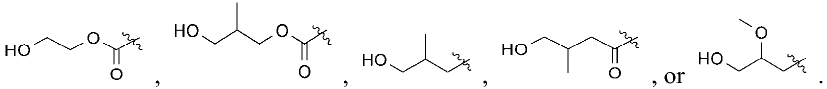

In some embodiments, in the PEG lipid represented by formula (II), R₁ is H-Z₁-Q₁-, Z₁ is -L₂-, Q₁ is -C(=O)-, and A₁ is a bond. In some such embodiments, L₂ is C₁~C₃ hydrocarbylene, preferably C₁~C₃ alkylene, more preferably C₁ alkylene or C₂ alkylene. In more preferred embodiments, R₁ is

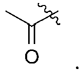

In some embodiments, the PEG lipid represented by formula (II) has a structure of 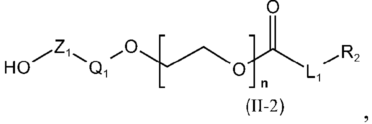 wherein Z₁, Q₁, L₁, R₂, and n are as defined in the formula (II), and Z₁ and Q₁ are not simultaneously a bond.

In some embodiments, in the PEG lipid represented by formula (II-2), Z₁ and Q₁ do not simultaneously contain -C(=O)-.

In some embodiments, in the PEG lipid represented by formula (II-2), Z₁ is -L₂-C(=O)-L₃-.

In some embodiments, the PEG lipid represented by formula (II) has a structure of 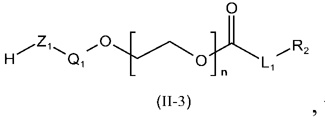 wherein Z₁, Q₁, L₁, R₂, and n are as defined in the formula (II), and when Q₁ is a bond, Z₁ is not a bond and is not an unsubstituted methylene.

In some embodiments, the PEG lipid represented by formula (II) has the a structure of 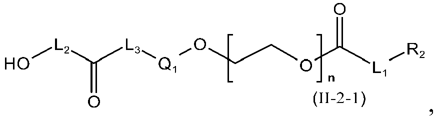 wherein L₁, L₂, L₃, Q₁, R₂, and n are as defined in the formula (II).

In some embodiments, the PEG lipid represented by formula (II) has a structure of 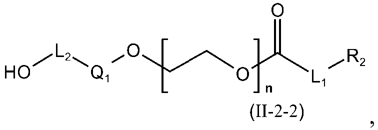 wherein L₁, L₂, Q₁, R₂, and n are as defined in the formula (II).

In some embodiments, the PEG lipid represented by formula (II) has a structure of 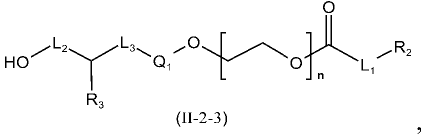 wherein L₁, L₂, L₃, Q₁, R₂, R₃, and n are as defined in the formula (II).

In some embodiments, the PEG lipid represented by formula (II) has a structure of 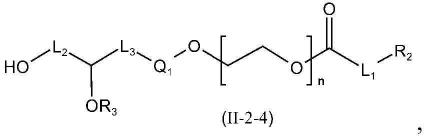 wherein L₁, L₂, L₃, Q₁, R₂, R₃, and n are as defined in the formula (II).

In some embodiments, the PEG lipid represented by formula (II) has a structure of 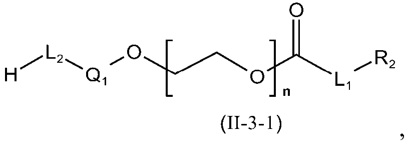 wherein L₁, L₂, Q₁, R₂, and n are as defined in the formula (II).

In some embodiments, in the PEG lipid represented by formula (II-2-1), Q₁ is -C(=O)-.

In some embodiments, in the PEG lipid represented by the formula (II-2-1), when Q₁ is - C(=O)-, L₃ is C₁ hydrocarbylene or C₄ hydrocarbylene.

In some embodiments, the PEG lipid represented by formula (II) has a structure of 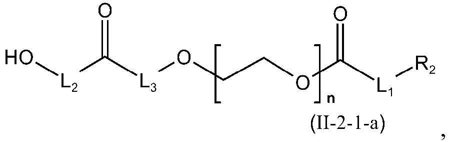 wherein L₁, L₂, L₃, R₂, and n are as defined in the formula (II).

In some embodiments, the PEG lipid represented by formula (II) has a structure of 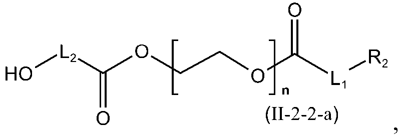 wherein L₁, L₂, R₂, and n are as defined in the formula (II).

In some embodiments, the PEG lipid represented by formula (II) has a structure of 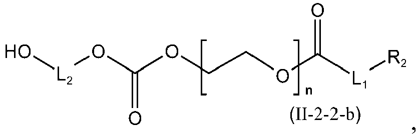 wherein L₁, L₂, R₂, and n are as defined in the formula (II).

In some embodiments, the PEG lipid represented by formula (II) has a structure of 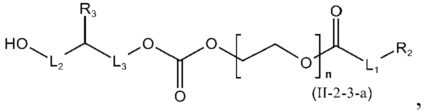 wherein L₁, L₂, L₃, R₂, R₃, and n are as defined in the formula (II).

In some embodiments, the PEG lipid represented by formula (II) has a structure of 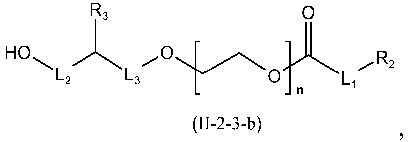 wherein L₁, L₂, L₃, R₂, R₃, and n are as defined in the formula (II).

In some embodiments, the PEG lipid represented by formula (II) has a structure of 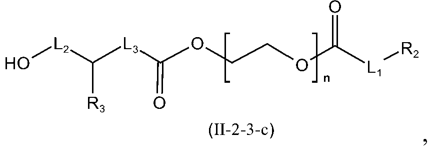 wherein L₁, L₂, L₃, R₂, R₃, and n are as defined in the formula (II).

In some embodiments, the PEG lipid represented by formula (II) has a structure of 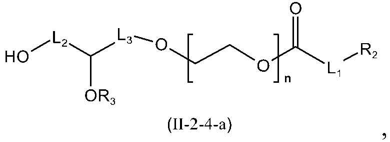 wherein L₁, L₂, L₃, R₂, R₃, and n are as defined in the formula (II).

In some embodiments, the PEG lipid represented by formula (II) has a structure of 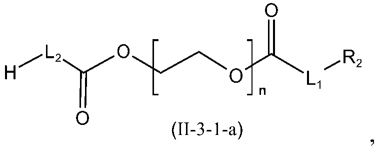 wherein L₁, L₂, R₂, and n are as defined in the formula (II).

In some embodiments, the PEG lipid represented by formula (II) has a structure of 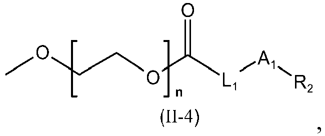 wherein,
R₂ and L₁ are as defined in the formula (II),
(i) A₁ is a bond, and R₂ and n satisfy the following conditions: (1) the total number of carbon atoms in L₁ and R₂ is 5~12, 10~12, 14~16, 18, or 20~32; and (2) n is any integer from 36 to 54; or
(ii) A₁ is -C(=O)O-, -OC(=O)-, -OC(=O)O-, -O- or -S-.

In some embodiments, in the PEG lipid represented by formula (II-4), A₁ is -C(=O)O-, - OC(=O)-, -OC(=O)O-, -O- or -S-, R₂ and n are as defined in the formula (I), and one or more of the following conditions are satisfied: (1) R₂ is C₄~C₁₇ hydrocarbyl or C₁₉~C₂₄ hydrocarbyl; and (2) the total number of the carbon atoms in the backbone of L₁ and the carbon atoms of R₂ is 5~19 or 21~32.

In some embodiments, in the PEG lipid represented by formula (III), R₁ is H or HO-Z₁-Q₁-, wherein Z₁ and Q₁ are as defined in the formula (I), and Z₁ and Q₁ are not simultaneously a bond, and A₁ is -C(=O)O-, -OC(=O)-, -OC(=O)O-, -O-, or a bond.

In some embodiments, the PEG lipid represented by formula (III) has a structure of 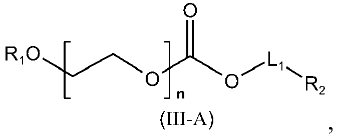 wherein R₁, L₁, R₂, and n are as defined in the formula (III).

In some embodiments, in the PEG lipid represented by the formula (III), R₁ is H or HO-Z₁-Q₁-, wherein Z₁ is -L₂-, Q₁ is -OC(=O)-, and A₁ is -C(=O)O-, -OC(=O)-, -OC(=O)O-, -O-, or a bond.

In some embodiments, the PEG lipid represented by formula (III) has a structure of 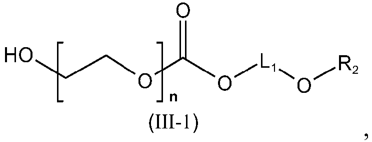 wherein L₁, R₂, and n are as defined in the formula (III).

In some embodiments, the PEG lipid represented by formula (III) has a structure of 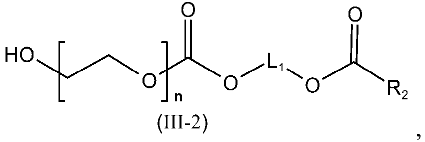 wherein L₁, R₂, and n are as defined in the formula (III).

In some embodiments, the PEG lipid represented by formula (III) has a structure of 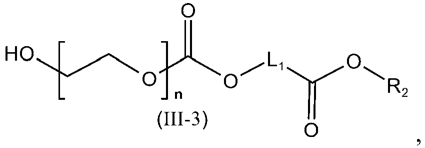 wherein L₁, R₂, and n are as defined in the formula (III).

In some embodiments, the PEG lipid represented by formula (III) has a structure of 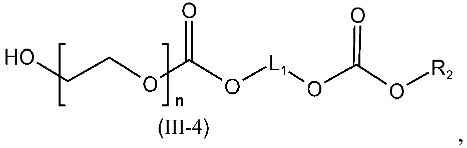 wherein L₁, R₂, and n are as defined in the formula (III).

In some embodiments, the PEG lipid represented by formula (III) has a structure of 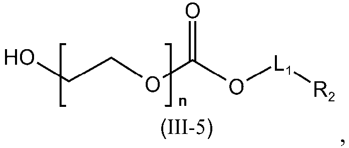 wherein L₁, R₂, and n are as defined in the formula (III).

In some embodiments, the PEG lipid represented by formula (III) has a structure of 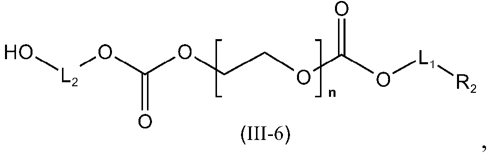 wherein L₁, L₂, R₂, and n are as defined in the formula (III).

In some embodiments, in the PEG lipid represented by formula (IV), formula (V) or formula (VI), R₁ is H, and A₁ is a bond, -C(=O)O-, -OC(=O)-, -OC(=O)O-, -O- or -S-.

In some embodiments, in the PEG lipid represented by formula (IV), formula (V) or formula (VI), R₁ is HO-Z₁-Q₁- or H-Z₁-Q₁-, and A₁ is a bond, -C(=O)O-, -OC(=O)- or -O-.

In some embodiments, the PEG lipid represented by formula (IV) has a structure of 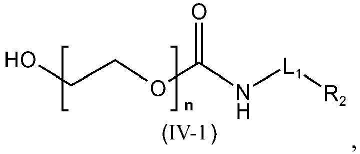 wherein L₁, R₂, and n are as defined in the formula (IV).

In some embodiments, the PEG lipid represented by formula (V) has a structure of 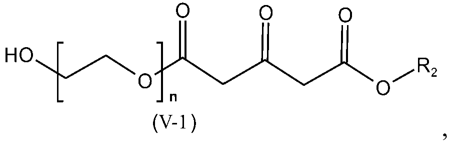 wherein R₂ and n are as defined in the formula (V).

In some embodiments, the PEG lipid represented by formula (VI) has a structure of 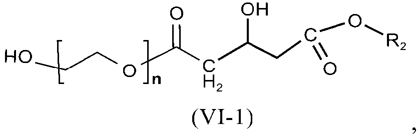 wherein R₂ and n are as defined in the formula (VI).

The PEG lipid represented by formula (VII) includes, when applicable, one or more of the following features.

In some embodiments, R₁ is HO-Z₁-Q₁- or H-Z₁-Q₁-, and A₁ is a bond, -C(=O)O-, - OC(=O)- or -O-.

In some embodiments, R₁ is HO-Z₁-Q₁- or H-Z₁-Q₁-, and Q₂ and Q₃ are bonds.

In some embodiments, R₁ is HO-Z₁-Q₁- or H-Z₁-Q₁-, Q₂ and Q₃ are bonds, and A₁ is - OC(=O)-.

In some embodiments, R₁ is HO-Z₁-Q₁- or H-Z₁-Q₁-, Q₂ and Q₃ are bonds, A₁ is -OC(=O)-, and Z₁ is -L₂-CH(OR₃)-L₃-, -L₂-C(=O)-L₃- or -L₂-CH(R₃)-L₃-.

In some embodiments, R₁ is HO-Z₁-Q₁- or H-Z₁-Q₁-, Q₂ and Q₃ are bonds, A₁ is -OC(=O)-, Z₁ is -L₂-CH(OR₃)-L₃-, -L₂-C(=O)-L₃- or -L₂-CH(R₃)-L₃-, and L₁ is a bond or C₁ alkylene.

In some embodiments, R₁ is H, and A₁ is a bond, -C(=O)O-, -OC(=O)-, -OC(=O)O- or - O-.

In some embodiments, Q₂ and Q₃ are bonds, R₁ is H, and Z₂ is -L₂-CH(OR₃)-L₃-, -L₂-C(=O)-L₃- or -L₂-CH(R₃)-L₃-.

In some embodiments, the PEG lipid represented by formula (VII) has a structure of 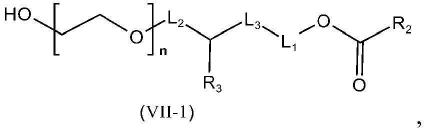 wherein L₁, L₂, L₃, R₂, R₃, and n are as defined in the formula (VII).

In some embodiments, the PEG lipid represented by formula (VII) has a structure of 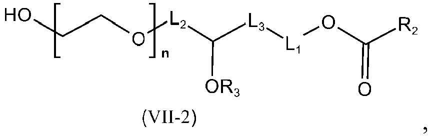 wherein L₁, L₂, L₃, R₂, R₃, and n are as defined in the formula (VII).

In some embodiments, the PEG lipid represented by formula (VII) has a structure of 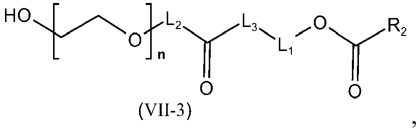 wherein L₁, L₂, L₃, R₂, and n are as defined in the formula (VII).

In some embodiments, in the PEG lipid represented by formula (I), Y is a bond, i.e., the PEG lipid represented by formula (I) has a structure of 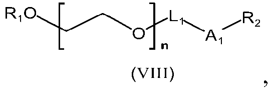 wherein R₁, A₁, L₁, R₂, and n are as defined in the formula (I), and
when R₁ is H and A₁ is -O-, then L₁ and R₂ satisfy at least one of the following conditions: (1) the total number of carbon atoms is not 16, (2) at least one of L₁ and R₂ has a branched chain, and (3) L₁ is C₃~C₈ hydrocarbylene, or a bond.

In some embodiments, A₁ of the PEG lipid represented by formula (VIII) is -C(=O)O-, - OC(=O)-, -OC(=O)O-, -S-, or a bond.

The PEG lipid represented by formula (I), formula (II), formula (II-1), formula (II-1-1), formula (II-2), formula (II-2-1), formula (II-2-1-a), formula (II-2-2), formula (II-2-2-a), formula (II-2-2-b), formula (II-2-3), formula (II-2-3-a), formula (II-2-3-b), formula (II-2-3-c), formula (II-2-4), formula (II-2-4-a), formula (II-3), formula (II-3-1), formula (II-3-1-a), formula (II-4), formula (III), formula (III-A), formula (III-1), formula (III-2), formula (III-3), formula (III-4), formula (III-5), formula (III-6), formula (IV), formula (IV-1), formula (V), formula (V-1), formula (VI), formula (VI-1), formula (VII), formula (VII-1), formula (VII-2), formula (VII-3), formula (VIII), formula (IX) or formula (X) includes, when applicable, one or more of the following features.

In some embodiments, the total number of carbon atoms in L₁ and R₂ is 12~25. For example, the total number of carbon atoms in L₁ and R₂ is 12, 13, 14, 15, 16, 17, 18, 19, 20, 21, 22, or 23.

In some embodiments, the total number of carbon atoms in L₁ and R₂ is 11~22.

In some embodiments, the total number of carbon atoms in L₁ and R₂ is 14~24.

In some embodiments, the total number of carbon atoms in L₁ and R₂ is 16~22.

In some embodiments, the total number of carbon atoms in L₁ and R₂ is 15~20.

In some embodiments, the total number of carbon atoms in L₁ and R₂ is 16~20.

In some embodiments, the total number of carbon atoms in L₁ and R₂ is 10~18.

In some embodiments, the total number of carbon atoms in L₁ and R₂ is 14~16. In some embodiments, L₁ is C₁~C₈ hydrocarbylene, or a bond.

In some embodiments, L₁ is C₁~C₂₀ alkylene, C₂~C₂₀ alkenylene or C₂~C₂₀ alkynylene.

In some embodiments, L₁ is C₁~C₈ alkylene, C₂~C₈ alkenylene or C₂~C₈ alkynylene.

In some embodiments, L₁ is substituted or unsubstituted C₁~C₂₀ hydrocarbylene, for example, substituted or unsubstituted C₁~C₁₅ hydrocarbylene, C₁~C₈ hydrocarbylene, C₄~C₈ hydrocarbylene, C₁~C₄ hydrocarbylene or C₁~C₂ hydrocarbylene.

In some embodiments, L₁ is substituted or unsubstituted C₁~C₂₀ alkylene, for example, substituted or unsubstituted C₁~C₁₅ alkylene, C₁~C₈ alkylene, C₄~C₈ alkylene, C₁~C₄ alkylene or C₁~C₂ alkylene.

In some embodiments, L₁ is straight or branched C₁~C₂₀ hydrocarbylene, for example, straight or branched C₁~C₁₅ hydrocarbylene, C₁~C₈ hydrocarbylene, C₄~C₈ hydrocarbylene, C₁~C₄ hydrocarbylene or C₁~C₂ hydrocarbylene.

In some embodiments, L₁ is straight or branched C₁~C₂₀ alkylene, for example, straight or branched C₁~C₁₅ alkylene, C₁~C₈ alkylene, C₄~C₈ alkylene, C₁~C₄ alkylene or C₁~C₂ alkylene.

In some embodiments, L₁ is straight C₁~C₂₀ alkylene, for example, straight C₁~C₁₅ alkylene, straight C₁~C₈ alkylene, straight C₄~C₈ alkylene, straight C₁~C₄ alkylene or straight C₁~C₂ alkylene.

In some embodiments, L₁ is a bond.

In some embodiments, R₂ is C₄~C₂₄ alkyl, C₄~C₂₄ alkenyl or C₄~C₂₄ alkynyl.

In some embodiments, R₂ is substituted or unsubstituted C₄~C₂₄ hydrocarbyl, for example, substituted or unsubstituted C₇~C₂₂ hydrocarbyl, C₁₁~C₂₂ hydrocarbyl, C₄~C₂₀ hydrocarbyl, C₈~C₂₀ hydrocarbyl, C₁₅~C₂₀ hydrocarbyl, C₁₂~C₁₈ hydrocarbyl, C₁₀~C₁₆ hydrocarbyl, C₄~C₁₅ hydrocarbyl, C₈~C₁₅ hydrocarbyl or C₅~C₁₁ hydrocarbyl.

In some embodiments, R₂ is substituted or unsubstituted C₄~C₂₄ alkyl, for example, substituted or unsubstituted C₇~C₂₂ alkyl, C₁₁~C₂₂ alkyl, C₄~C₂₀ alkyl, C₈~C₂₀ alkyl, C₁₅~C₂₀ alkyl, C₁₂~C₁₈ alkyl, C₁₀~C₁₆ alkyl, C₄~C₁₅ alkyl, C₈~C₁₅ alkyl or C₅~C₁₁ alkyl.

In some embodiments, R₂ is straight or branched C₄~C₂₄ hydrocarbyl, for example, straight or branched C₇~C₂₂ hydrocarbyl, C₁₁~C₂₂ hydrocarbyl, C₄~C₂₀ hydrocarbyl, C₈~C₂₀ hydrocarbyl, C₁₅~C₂₀ hydrocarbyl, C₁₂~C₁₈ hydrocarbyl, C₁₀~C₁₆ hydrocarbyl, C₄~C₁₅ hydrocarbyl, C₈~C₁₅ hydrocarbyl or C₅~C₁₁ hydrocarbyl.

In some embodiments, R₂ is straight or branched C₄~C₂₄ alkyl, for example, straight or branched C₇~C₂₂ alkyl, C₁₁~C₂₂ alkyl, C₄~C₂₀ alkyl, C₈~C₂₀ alkyl, C₁₅~C₂₀ alkyl, C₁₂~C₁₈ alkyl, C₁₀~C₁₆ alkyl, C₄~C₁₅ alkyl, C₈~C₁₅ alkyl or C₅~C₁₁ alkyl.

In some embodiments, R₂ is straight C₄~C₂₄ alkyl, for example, straight C₇~C₂₂ alkyl, straight C₁₁~C₂₂ alkyl, straight C₄~C₂₀ alkyl, straight C₈~C₂₀ alkyl, straight C₁₅~C₂₀ alkyl, straight C₁₂~C₁₈ alkyl, straight C₁₀~C₁₆ alkyl, straight C₄~C₁₅ alkyl, straight C₈~C₁₅ alkyl or straight C₅~C₁₁ alkyl.

In some embodiments, R₂ is C₇~C₂₂ hydrocarbyl.

In some embodiments, R₂ is C₇~C₂₂ alkyl, C₇~C₂₂ alkenyl or C₇~C₂₂ alkynyl.

In some embodiments, L₁ is straight C₁~C₈ alkylene, and R₂ is straight C₇~C₂₂ alkyl.

In some embodiments, -L₁-R₂ is straight C₁₆~C₂₂ hydrocarbyl.

In some embodiments, -L₁-R₂ is C₁₆~C₂₂ alkyl.

In some embodiments, -L₁-R₂ is straight C₁₆~C₂₂ alkyl.

In some embodiments, -L₁-R₂ is C₁₆~C₁₈ hydrocarbyl.

In some embodiments, -L₁-R₂ is straight C₁₆~C₁₈ hydrocarbyl.

In some embodiments, -L₁-R₂ is C₁₆~C₁₈ alkyl.

In some embodiments, -L₁-R₂ is straight C₁₆~C₁₈ alkyl.

In some embodiments, R₃ is H, C₁~C₃ alkyl, C₂~C₃ alkenyl or C₂~C₃ alkynyl.

In some embodiments, R₃ is H or C₁~C₃ alkyl.

In some embodiments, R₃ is C₁~C₃ alkyl, C₂~C₃ alkenyl or C₂~C₃ alkynyl.

In some embodiments, R₃ is C₁~C₂ hydrocarbyl.

In some embodiments, R₃ is C₁ hydrocarbyl or C₂ hydrocarbyl.

In some embodiments, R₃ is C₁ alkyl or C₂ alkyl.

In some embodiments, R₃ is H.

In some embodiments, R₃ is C₁ alkyl.

In some embodiments, R₃ is C₂ alkyl.

In some embodiments, n is any integer from 30~55, for example, n is any integer from 30~50, 34~50, 34~55, 36~55, 40~55, 44~55, 48~55, 50~55, 36~54, 40~54, 44~54, 48~54 or 50~54.

In some embodiments, n is any integer from 35~55.

In some embodiments, n is any integer from 35~50.

In some embodiments, n is any integer from 40~50.

In some embodiments, n is 45.

In some embodiments, L₂ is C₁~C₃ hydrocarbylene.

In some embodiments, L₂ is C₁~C₃ alkylene, C₂~C₃ alkenylene or C₂~C₃ alkynylene.

In some embodiments, L₂ is C₁~C₃ alkylene.

In some embodiments, L₂ is straight C₁~C₃ alkylene.

In some embodiments, L₂ is C₁ alkylene or C₂ alkylene.

In some embodiments, L₃ is C₁~C₂ hydrocarbylene.

In some embodiments, L₃ is C₁~C₂ alkylene, C₂ alkenylene or C₂ alkynylene.

In some embodiments, L₃ is C₁~C₂ alkylene.

In some embodiments, the PEG lipid represented by formula (I) has a structure as shown in Table 1 below:

**Table 1**

| No. | Structure |
|---|---|
| 001 | 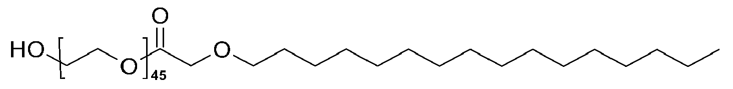 |
| 002 | 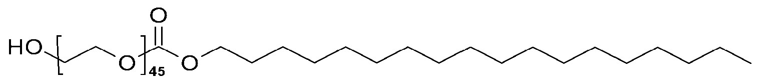 |
| 003 | 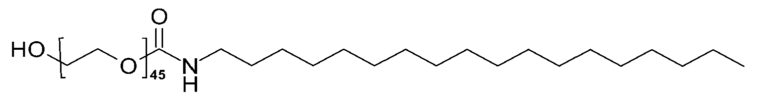 |
| 004 | 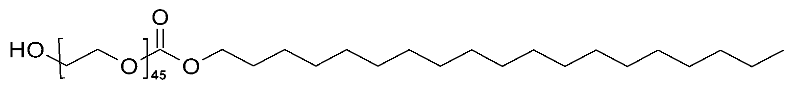 |
| 005 | 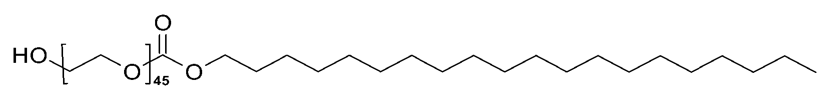 |
| 006 | 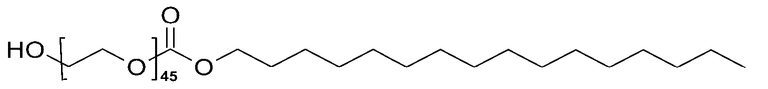 |
| 007 | 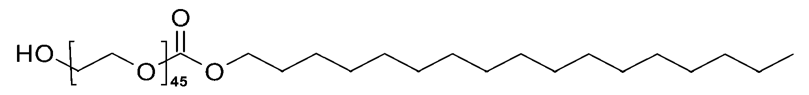 |
| 008 | 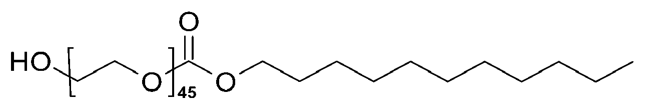 |
| 009 | 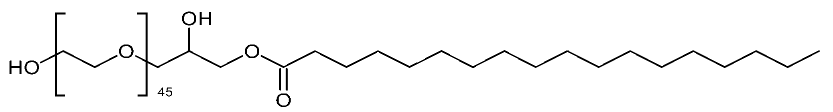 |
| 010 | 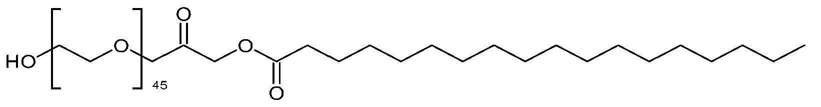 |
| 011 | 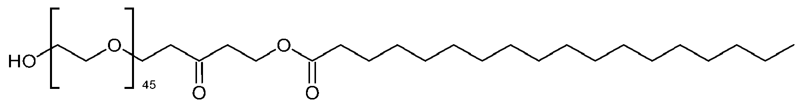 |
| 012 | 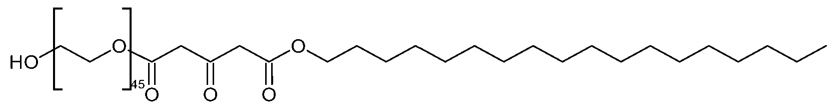 |
| 013 | 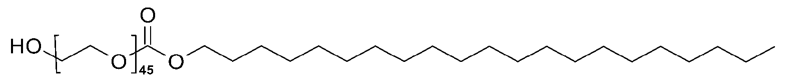 |
| 014 | 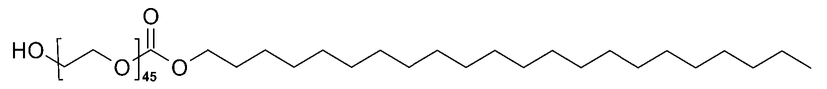 |
| 015 | 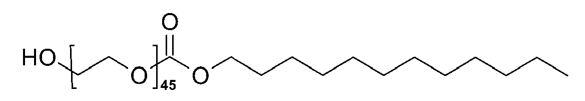 |
| 016 | 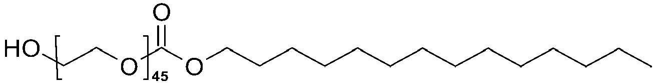 |
| 017 | 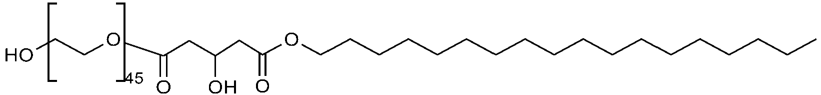 |
| 018 | 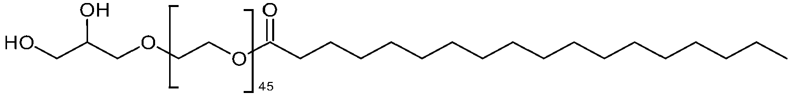 |
| 019 | 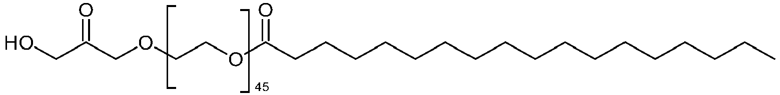 |
| 020 | 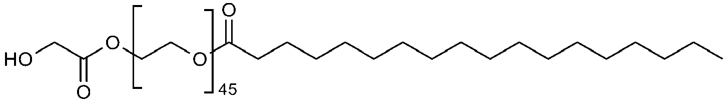 |
| 021 | 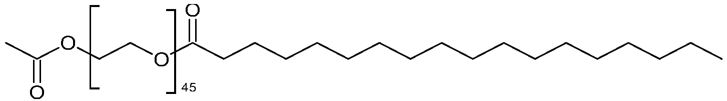 |
| 022 | 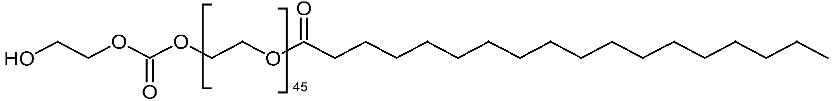 |
| 023 | 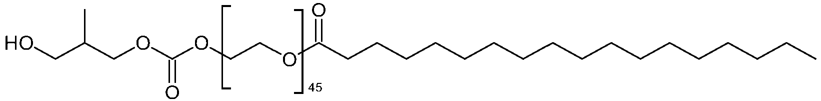 |
| 024 | 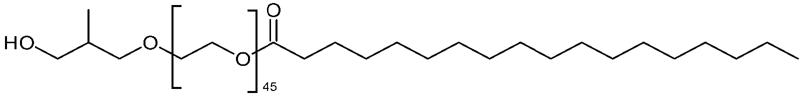 |
| 025 | 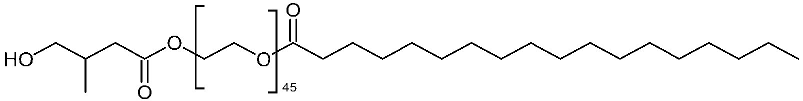 |
| 026 | 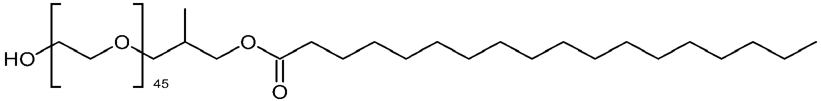 |
| 027 | 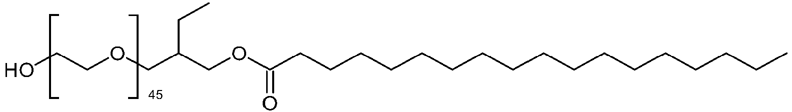 |
| 028 | 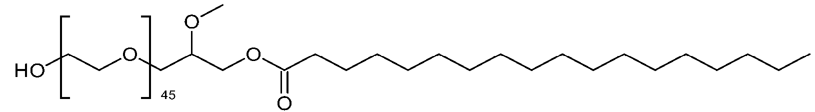 |
| 029 | 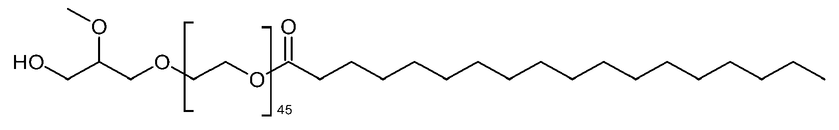 |
| 030 | 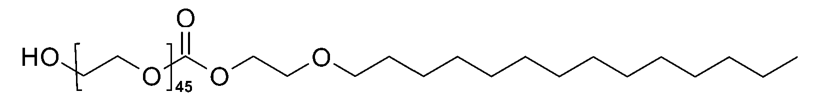 |
| 031 | 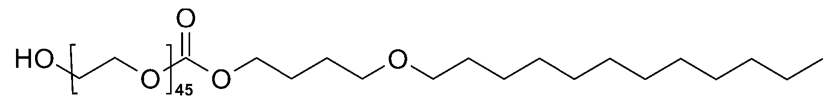 |
| 032 | 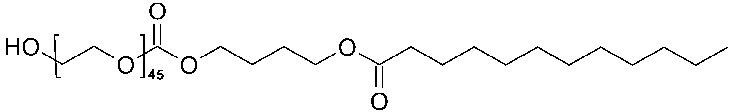 |
| 033 | 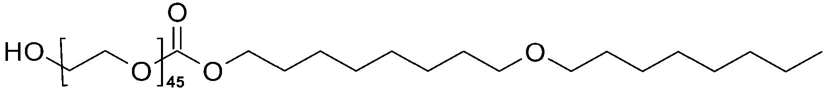 |
| 034 | 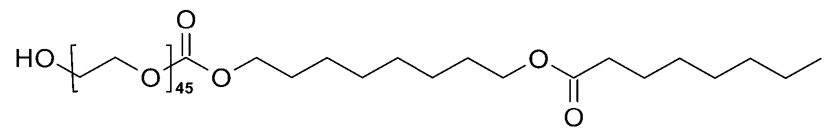 |
| 035 | 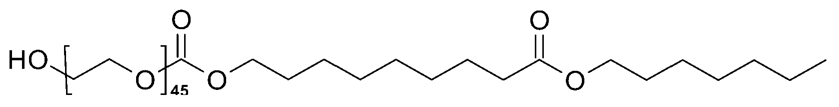 |
| 036 | 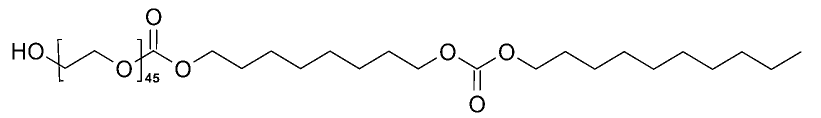 |
| 037 | 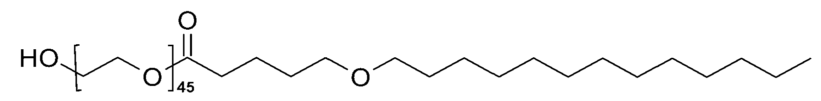 |
| 038 | 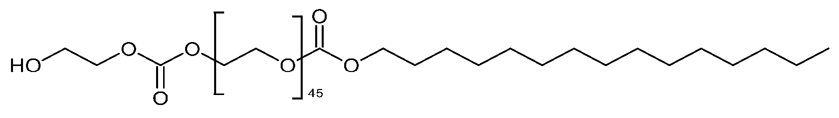 |

### III. LIPID NANOPARTICLES, PHARMACEUTICAL COMPOSITIONS, MEDICAMENTS, AND USES THEREOF

The present disclosure also provides a lipid nanoparticle comprising the PEG lipid according to any of the preceding embodiments.

In some embodiments, the lipid nanoparticle described above refers to a particle having a nanometer-scale size (e.g., 1 nm~1000 nm).

In some embodiments, the lipid nanoparticle described above has an average diameter of 20 nm~800 nm, 20 nm~500 nm, 20 nm~400 nm, 20 nm~300 nm, 20 nm~200 nm, 20 nm~100 nm, 30 nm~700 nm, 30 nm~500 nm, 30 nm~300 nm, 30 nm~200 nm, 30 nm~100 nm, 40 nm~800 nm, 40 nm~600 nm, 40 nm~500 nm, 40 nm~300 nm, 40 nm~200 nm, 40 nm~100 nm, 50 nm~800 nm, 50 nm~600 nm, 50 nm~500 nm, 50 nm~400 nm, 50 nm~300 nm, 50 nm~200 nm, 50 nm~100 nm, 60 nm~800 nm, 60 nm~600 nm, 60 nm~500 nm, 60 nm~400 nm, 60 nm~300 nm, 60 nm~200 nm, or 60 nm~100 nm. In some optional specific examples, the lipid nanoparticle described above has an average diameter of 26 nm, 31 nm, 36 nm, 41 nm, 46 nm, 51 nm, 56 nm, 61 nm, 66 nm, 71 nm, 76 nm, 81 nm, 86 nm, 91 nm, 96 nm, 101 nm, 106 nm, 111 nm, 116 nm, 121 nm, 126 nm, 131 nm, 136 nm, 141 nm, 146 nm, 151 nm, 156 nm, 161 nm, 166 nm, 171 nm, 176 nm, 181 nm, 186 nm, 191 nm, 196 nm, 201 nm, 206 nm, 211 nm, 216 nm, 221 nm, 226 nm, 231 nm, 236 nm, 241 nm, 246 nm, or 249 nm. Herein, the average diameter of lipid nanoparticles can be expressed as the z-average determined by dynamic light scattering.

In some embodiments, the lipid nanoparticle described above includes one of cationic lipid nanoparticles, solid lipid nanoparticles (SLN), nanostructured lipid carriers (NLC), or nonlamellar lipid nanoparticles. In one alternative specific example, the lipid nanoparticle described above is a cationic lipid nanoparticle.

In some embodiments, the lipid nanoparticle described above further contains one or more of the following substances: a cationic lipid, a helper lipid, and a structural lipid.

In some embodiments, the cationic lipid includes a compound represented by formula (XI): 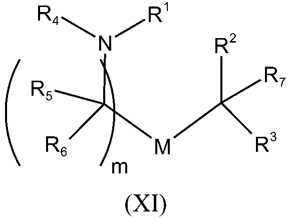 an N-oxide thereof, a salt thereof or an isomer thereof,
wherein,
R¹ is selected from the group consisting of C₅~C₃₀ alkyl, C₅~C₂₀ alkenyl, -R*Y₁R", -Y₁R" and -R"'M'R';
R² and R³ are independently selected from the group consisting of H, C₁~C₁₄ alkyl, C₂~C₁₄ alkenyl, -R*Y₁R", -Y₁R" and -R*OR", or R² and R³ together with the atom to which they are attached form a heterocycle or a carbocycle;
R₄ is selected from the group consisting of hydrogen, C₃~C₆ carbocycle, -(CH₂)ₙQ, - (CH₂)ₙCHQR, -(CH₂)ₒC(R₁₀)₂(CH₂)ₙ₋ₒQ, -CHQR, -CQ(R)₂ and unsubstituted C₁~C₆ alkyl, wherein Q is selected from the group consisting of a carbocycle, a heterocycle, -OR, - O(CH₂)ₙN(R)₂, -C(O)OR, -OC(O)R, -CX₃, -CX₂H, -CXH₂, -CN, -N(R)₂, -C(O)N(R)₂, - N(R)C(O)R, -N(R)S(O)₂R, -N(R)C(O)N(R)₂, -N(R)C(S)N(R)₂, N(R)R₈, -N(R)S(O)₂R₈, - O(CH₂)ₙOR, -N(R)C(=NR₉)N(R)₂, -N(R)C(=CHR₉)N(R)₂, -OC(O)N(R)₂, -N(R)C(O)OR, - N(OR)C(O)R, -N(OR)S(O)₂R, -N(OR)C(O)OR, -N(OR)C(O)N(R)₂, -N(OR)C(S)N(R)₂, - N(OR)C(=NR₉)N(R)₂, -N(OR)C(=CHR₉)N(R)₂, -C(=NR₉)N(R)₂, -C(=NR₉)R, -C(O)N(R)OR and -C(R)N(R)₂C(O)OR, each o is independently selected from 1, 2, 3 and 4, and each n is independently selected from 1, 2, 3, 4 and 5;
each R₅ is independently selected from the group consisting of OH, C₁~C₃ alkyl, C₂~C₃ alkenyl and H;
each R₆ is independently selected from the group consisting of OH, C₁~C₃ alkyl, C₂~C₃ alkenyl and H;
M and M' are independently selected from the group consisting of -C(O)O-, -OC(O)-, - OC(O)-M"C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, an aryl group and a heteroaryl group, wherein M" is a bond, C₁~C₁₃ alkylene or C₂~C₁₃ alkenylene;
R₇ is selected from the group consisting of C₁~₃ alkyl, C₂~C₃ alkenyl and H;
R₈ is selected from the group consisting of C₃~₆ carbocycle and heterocycle;
R₉ is selected from the group consisting of H, CN, NO₂, C₁~C₆ alkyl, -OR, -S(O)₂R, - S(O)₂N(R)₂, C₂~C₆ alkenyl, C₃~C₆ carbocycle and heterocycle;
R₁₀ is selected from the group consisting of H, C₁~C₃ alkyl and C₂~C₃ alkenyl;
each R is independently selected from the group consisting of C₁~C₃ alkyl, C₂~C₃ alkenyl, (CH₂)_{q}OR** and H,
and each q is independently selected from 1, 2 and 3;
each R' is independently selected from the group consisting of C₁~C₁₈ alkyl, C₂~C₁₈ alkenyl, -R*Y₁R", -Y₁R", H and 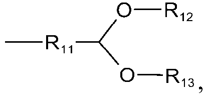 and R₁₁ is selected from the group consisting of C₁~C₁₂ alkylene and C₂~C₁₂ alkenylene, R₁₂ and R₁₃ are each independently selected from the group consisting of C₁~C₁₂ alkyl and C₂~C₁₂ alkenyl;
each R" is independently selected from the group consisting of C₃~C₁₅ alkyl and C₃~C₁₅ alkenyl;
each R‴ is independently selected from the group consisting of C₃~C₁₅ alkylene and C₃~C₁₅ alkenylene;
each R* is independently selected from the group consisting of absent, C₁~C₁₂ alkylene and C₂~C₁₂ alkenylene;
each R** is independently selected from the group consisting of absent, C₁~C₁₂ alkyl and C₂~C₁₂ alkenyl;
each Y₁ is independently a C₃~C₆ carbocycle;
each X is independently selected from the group consisting of F, Cl, Br and I; and
m is selected from 5, 6, 7, 8, 9, 10, 11, 12 and 13; and
wherein when R₄ is -(CH₂)ₙQ, -(CH₂)ₙCHQR, -CHQR or -CQ(R)₂, then (i) when n is 1, 2, 3, 4 or 5, Q is not -N(R)₂; or (ii) when n is 1 or 2, Q is not a 5-, 6- or 7-membered heterocycloalkyl.

In one alternative specific example, the cationic lipid is the compound represented by formula (XI): 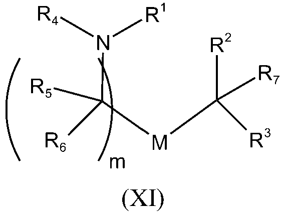 an N-oxide thereof, a salt thereof or an isomer thereof,
wherein, R¹~R³, R₄~R₇, M and m are as defined in the formula (XI) above.

In one alternative specific example, the cationic lipid is a compound represented by formula (XI-1): 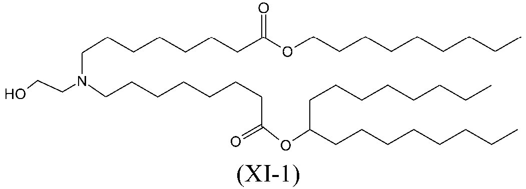 an N-oxide thereof, a salt thereof or an isomer thereof.

In some embodiments, the cationic lipid includes a compound represented by formula (XII): 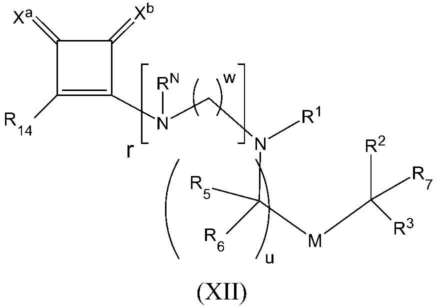 an N-oxide thereof, a salt thereof or an isomer thereof,
wherein,
R¹, R², R³, R₅, R₆, M and R₇ are as defined in formula (XI) above,
R^{N} is H or C₁~C₃ alkyl;
X^{a} and X^{b} are each independently O or S;
R₁₄ is selected from the group consisting of H, halogen, -OH, R^{b}, -N(R^{b})₂, -CN, -N₃, - C(O)OH, -C(O)OR^{b}, -OC(O)R^{b}, -OR^{b}, -SR^{b}, -S(O)R^{b}, -S(O)OR^{b}, -S(O)₂OR^{b}, -NO₂, - S(O)₂N(R^{b})₂, -N(R^{b})S(O)₂R^{b}, -NH(CH₂)ₜ₁N(R^{b})₂, -NH(CH₂)ₚ₁O(CH₂)_{q1}N(R^{b})₂, - NH(CH₂)ₛ₁OR^{b}, -N((CH₂)ₛ₁OR^{b})₂, -N(R^{b})-carbocycle, -N(R^{b})-heterocycle, -N(R^{b})-aryl, - N(R^{b})-heteroaryl, -N(R^{b})(CH₂)ₜ₁-carbocycle, -N(R^{b})(CH₂)ₜ₁-heterocycle, -N(R^{b})(CH₂)ₜ₁-aryl, - N(R^{b})(CH₂)ₜ₁-heteroaryl, carbocycle, heterocycle, aryl and heteroaryl;
each R^{b} is independently selected from the group consisting of C₁~C₃ alkyl, C₂~C₃ alkenyl and H;
u is 5, 6, 7, 8, 9, 10, 11, 12 or 13;
w is 1, 2, 3, 4, 5, 6, 7, 8, 9 or 10;
r is 0 or 1;
t1 is 1, 2, 3, 4 or 5;
p1 is 1, 2, 3, 4 or 5;
q1 is 1, 2, 3, 4 or 5; and
s1 is 1, 2, 3, 4 or 5.

In one alternative specific example, the cationic lipid is the compound represented by formula (XII): 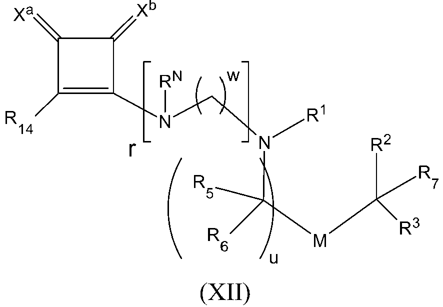 an N-oxide thereof, a salt thereof or an isomer thereof,
wherein, R¹~R³, R₅~R₇, R₁₄, X^{a}, X^{b}, R^{N}, M, u, w and r are as defined in the formula (XII) above.

In some embodiments, the cationic lipid includes a compound represented by formula (XIII): 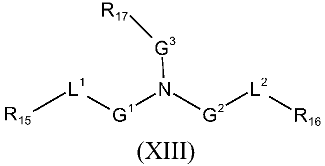 an N-oxide thereof, a salt thereof or an isomer thereof,
wherein:
one of L¹ or L² is -O(C=O)-, -(C=O)O-, -C(=O)-, -O-, -S(O)ₓ-, -S-S-, -C(=O)S-, -SC(=O)-, -NR^{a}C(=O)-, -C(=O)NR^{a}-, -NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, -SC(=O)-, - NR^{a}C(=O)-, -C(=O)NR^{a}-, -NR^{a}C(=O)NR^{a}-, -OC(=O)NR^{a}- or -NR^{a}C(=O)O-, or a bond;
G¹ and G² are each independently unsubstituted C₁~C₁₂ alkylene or C₂~C₁₂ alkenylene;
G³ is C₁~C₂₄ alkylene, C₂~C₂₄ alkenylene, C₃~C₈ cycloalkylene, C₃~C₈ cycloalkenylene;
R^{a} is H or C₁~C₁₂ hydrocarbyl;
R₁₅ and R₁₆ are each independently C₆~C₂₄ alkyl or C₆~C₂₄ alkenyl;
R₁₇ is H, OR₁₈, CN, -C(=O)OR₁₉, -OC(=O)R₁₉ or -NR₁₅C(=O)R₁₉;
R₁₉ is C₁~C₁₂ hydrocarbyl;
R₁₈ is H or C₁~C₆ hydrocarbyl; and
x is 0, 1 or 2.

In one alternative specific example, the cationic lipid is the compound represented by formula (XIII): 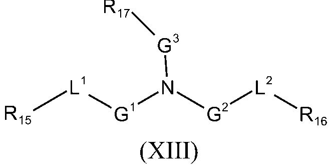 an N-oxide thereof, a salt thereof or an isomer thereof,
wherein: R₁₅~R₁₇, G¹~G³, and L¹~L² are as defined in the formula (XIII) above.

In some embodiments, the cationic lipid includes a compound represented by formula (XIV): 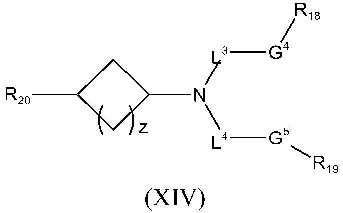 or a pharmaceutically acceptable salt or stereoisomer thereof,
wherein:
L³ and L⁴ which are the same or different are each independently C₁~C₁₂ alkylene, C₂~C₁₂ alkenylene or C₂~C₁₂ alkynylene; in some embodiments L³ and L⁴ which are the same or different are each independently C₃~C₁₀ alkylene, C₃~C₁₀ alkenylene or C₃~C₁₀ alkynylene; in some embodiments, L³ and L⁴ which are the same or different are each independently C₃~C₁₀ alkylene; in some embodiments, L³ and L⁴ which are the same or different are each independently C₅~C₈ alkylene;
G⁴ and G⁵ which are the same or different are each independently -O-(C=O)-, -(C=O)-O-, -C(=O)-, -O-, -C(=O)-S- or -S-C(=O)-; in some embodiments, G⁴ and G⁵ which are the same or different are each independently -O-(C=O)-, -(C=O)-O-, -C(=O)- or -O-; in some embodiments, G⁴ and G⁵ which are the same or different are each independently selected from -O-(C=O)- or -(C=O)-O-;
R₁₈ and R₁₉ which are the same or different are each independently C₅~C₂₇ alkyl, or C₅~C₂₇ alkenyl containing one or more double bonds; in some embodiments, R₁₈ and R₁₉ which are the same or different are each independently C₈~C₂₀ alkyl, or C₈~C₂₀ alkenyl containing one or more double bonds; in some embodiments, R₁₈ and R₁₉ which are the same or different are each independently C₉~C₁₇ alkyl, or C₉~C₁₈ alkenyl containing one or two double bonds; in some embodiments, R₁₈ and R₁₉ which are the same or different are each independently 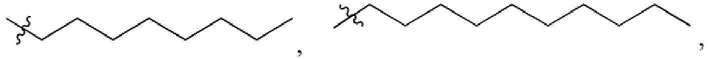 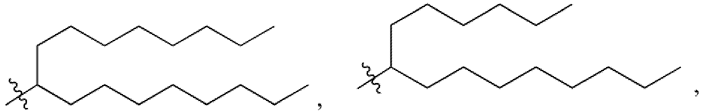 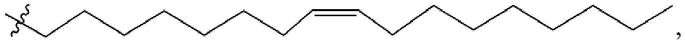 or 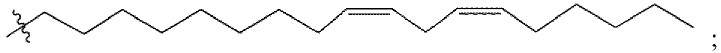
R₂₀ is halogen, hydroxy, cyano, C₁~C₆ alkyl, nitro, C₁~C₆ alkoxy, C₁~C₆ alkylcarbonyloxy, C₁~C₆ alkoxycarbonyl, C₁~C₆ alkylaminocarbonyl or C₁~C₆ alkylcarbonylamino; in some embodiments, R₂₀ is halogen, hydroxy, cyano, C₁~C₆ alkoxy, C₁~C₆ alkylcarbonyloxy, C₁~C₆ alkoxycarbonyl, C₁~C₆ alkylaminocarbonyl or C₁~C₆ alkylcarbonylamino; in some embodiments, R₂₀ is halogen, hydroxy, cyano, C₁~C₄ alkoxy, C₁~C₄ alkylcarbonyloxy, C₁~C₄ alkoxycarbonyl, C₁~C₄ alkylaminocarbonyl or C₁~C₄ alkylcarbonylamino; in some embodiments, R₂₀ is fluorine, hydroxy, cyano, methoxy, acetoxy, methoxycarbonyl, butylaminocarbonyl or acetylamino;
z is 1, 2 or 3.

In some embodiments, the cationic lipid is the compound represented by formula (XIV): 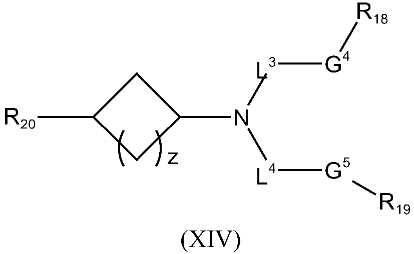 or a pharmaceutically acceptable salt or stereoisomer thereof,
wherein: R₁₈~R₂₀, G⁴~G⁵, L³~L⁴ and z are as defined in the formula (XIV) above.

In some embodiments, the cationic lipid includes compound (XIV-1) below: 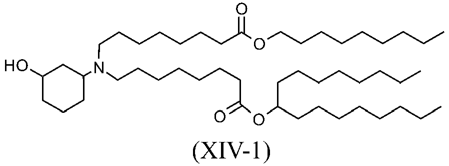 or a pharmaceutically acceptable salt or a stereoisomer thereof.

In some embodiments, the cationic lipid includes a compound selected from the group consisting of: 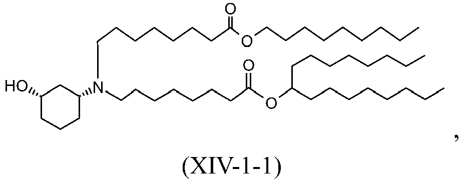 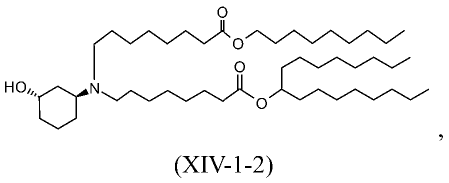 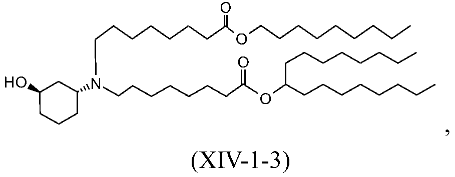 or 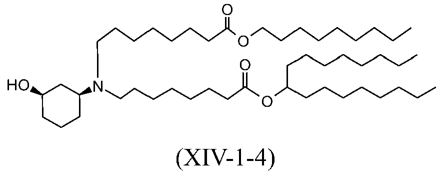 or a pharmaceutically acceptable salt thereof.

In some embodiments, the cationic lipid of the lipid nanoparticle described above includes one or more of ALC-0315 (CAS No. 2036272-55-4), SM-102 (CAS No. 2089251-47-6) and

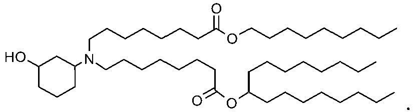

In some embodiments, the helper lipid of the lipid nanoparticle described above includes a phospholipid. Phospholipids usually semi-synthetic, but can also be of natural origin or chemically modified. In one alternative specific example, the helper lipid of the lipid nanoparticle described above is a phospholipid. In some embodiments, the phospholipid of the lipid nanoparticle described above includes one or more of DSPC (distearoyl phosphatidylcholine), DOPE (dioleoyl phosphatidylethanolamine), DOPC (dioleoyl phosphatidylcholine), DOPS (dioleoyl phosphatidylserine), DSPG (1,2-distearoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl)homoserine) and lysophospholipid. In some embodiments, the helper lipid of the lipid nanoparticle described above is selected from one or more of DSPC, DOPE, DOPC and DOPS. In some embodiments, the helper lipid of the lipid nanoparticle described above is DSPC and/or DOPE.

In some embodiments, the structural lipid of the lipid nanoparticle described above includes a sterol. In one alternative specific example, the structural lipid of the lipid nanoparticle described above is a sterol. In some embodiments, the sterol of the lipid nanoparticle described above includes one or more of 20α-hydroxycholesterol, cholesterol, a cholesterol ester, a steroid hormone, a steroid vitamin, a bile acid, cholesterin, ergosterol, β-sitosterol and an oxidized derivative of cholesterol. In some embodiments, the structural lipid of the lipid nanoparticle described above includes at least one of cholesterol, a cholesterol ester, a steroid hormone, a steroid vitamin and a bile acid. In some embodiments, the structural lipid of the lipid nanoparticle described above is cholesterol. In one alternative specific example, the structural lipid of the lipid nanoparticle described above is high-purity cholesterol, especially injection-grade high-purity cholesterol, for example, CHO-HP (produced by AVT). In other embodiments, the structural lipid is 20α-hydroxycholesterol.

In some embodiments, the lipid nanoparticle described above contains a cationic lipid, a helper lipid, a structural lipid, and the PEG lipid according to any of the preceding embodiments. In some embodiments, the lipid nanoparticle described above comprises about 25%~75%, for example, about 25%~28%, 28%~32%, 32%~35%, 35%~40%, 40%~42%, 42%~45%, 45%~46.3%, 46.3%~48%, 48%~49.5%, 49.5%~50%, 50%~55%, 55%~60%, 60%~65% or 65%~75% (mole percentage) of the cationic lipid, based on the total amount of the cationic lipid, the helper lipid, the structural lipid and the PEG lipid.

In some embodiments, the lipid nanoparticle described above comprises about 5%~45%, for example, about 5%~9%, 9%~9.4%, 9.4%~10%, 10%~10.5%, 10.5%~11%, 11%~15%, 15%~16%, 16%~18%, 18%~20%, 20%~25%, 25%~33.5%, 33.5%~37%, 37%~40%, 40%~42% or 42%~45% (mole percentage) of the helper lipid, based on the total amount of the cationic lipid, the helper lipid, the structural lipid and the PEG lipid.

In some embodiments, the lipid nanoparticle described above comprises about 0%~50%, for example, about 0%~10%, 10%~15.5%, 15.5%~18.5%, 18.5%~22.5%, 22.5%~23.5%, 23.5%~28.5%, 28.5%~33.5%, 33.5%~35%, 35%~36.5%, 36.5%~38%, 38%~38.5%, 38.5%~39%, 39%~39.5%, 39.5%~40.5%, 40.5%~41.5%, 41.5%~42.5%, 42.5%~42.7%, 42.7%~43%, 43%~43.5%, 43.5%~45%, 45%~46.5%, 46.5%~48.5% or 46.5%~50% (mole percentage) of the structural lipid, based on the total amount of the cationic lipid, the helper lipid, the structural lipid and the PEG lipid.

In some embodiments, the lipid nanoparticle described above comprises about 0.5%~5%, for example, about 0.5%~1%, 1%~1.5%, 1.5%~1.6%, 1.6%~2%, 2%~2.5%, 2.5%~3%, 3%~3.5%, 3.5%~4%, 4%~4.5% or 4.5%~5% (mole percentage) of the PEG lipid, based on the total amount of the cationic lipid, the helper lipid, the structural lipid and the PEG lipid.

In some embodiments as described above, the lipid nanoparticle has a molar ratio of cationic lipid:helper lipid: structural lipid:PEG lipid at about 45:10:42.5:2.5, 45:11:41.5:2.5, 42:10.5:45:2.5, 42:16:39.5:2.5, 40:16:41.5:2.5, 40:18:39.5:2.5, 35:16:46.5:2.5, 35:25:36.5:3.5, 28:33.5:35:3.5, 32:37:40.5:0.5, 35:40:22.5:2.5, 40:42:15.5:2.5, 40:20:38.5:1.5, 45:15:38.5:1.5, 55:5:38.5:1.5, 60:5:33.5:1.5, 45:20:33.5:1.5, 50:20:28.5:1.5, 55:20:23.5:1.5, 60:20:18.5:1.5, 40:15:43.5:1.5, 50:15:33.5:1.5, 55:15:28.5:1.5, 60:15:23.5:1.5, 40:10:48.5:1.5, 45:10:43.5:1.5, 55:10:33.5:1.5, 40:5:53.5:1.5, 45:5:48.5:1.5, or 50:5:43.5:1.5. In some such embodiments, the helper lipid is DOPE, and the structural lipid is CHO-HP.

In other embodiments as described above, the lipid nanoparticle has a molar ratio of cationic lipid:helper lipid: structural lipid:PEG lipid at about 50:10:38.5:1.5, 50:9:38:3, 49.5:10:39:1.5, 48:10:40.5:1.5, 46.3:9.4:42.7:1.6, 45:9:43:3, 45:11:41.5:2.5, 42:10.5:45:2.5, 42:16:39.5:2.5, 40:16:41.5:2.5, 40:18:39.5:2.5, 35:40:22.5:2.5, 40:20:38.5:1.5, 45:15:38.5:1.5, 55:5:38.5:1.5, 60:5:33.5:1.5, 45:20:33.5:1.5, 50:20:28.5:1.5, 55:20:23.5:1.5, 60:20:18.5:1.5, 40:15:43.5:1.5, 50:15:33.5:1.5, 55:15:28.5:1.5, 60:15:23.5:1.5, 40:10:48.5:1.5, 45:10:43.5:1.5, 55:10:33.5:1.5, 40:5:53.5:1.5, 45:5:48.5:1.5, or 50:5:43.5:1.5. In some such embodiments, the helper lipid is DSPC, and the structural lipid is CHO-HP.

In some embodiments, the lipid nanoparticle described above further comprises an active ingredient.

In some embodiments, the active ingredient is a pharmaceutically active ingredient.

In some embodiments, the active ingredient is a nucleic acid, for example, DNA, or RNA. In some embodiments, the pharmaceutically active ingredient is a nucleic acid, for example, DNA, or RNA. In some embodiments, the pharmaceutically active ingredient is one or more of RNA and DNA. In some embodiments, the RNA is one or more of mRNA, antisense oligonucleotide, ribosomal RNA (rRNA), microRNA (miRNA), transfer RNA (tRNA), small interfering RNA (siRNA), small nuclear RNA (snRNA), small hairpin RNA (shRNA), single-guide RNA (sgRNA) and Cas9 mRNA. In some embodiments, the pharmaceutically active ingredient is mRNA. In some embodiments, the pharmaceutically active ingredient is a plasmid.

In some embodiments, the nucleic acid serving as the pharmaceutically active ingredient encodes a polypeptide and/or protein of interest. The polypeptide or protein of interest refers to a therapeutically or pharmaceutically active polypeptide or protein having a therapeutic or preventive effect, whose function in or near cells is required or beneficial, for example, a protein of which the deficiency or a defective form leads to the occurrence of a disease and of which the provision can modulate or prevent the disease, or a protein of which the presence in or near cells is beneficial to the body. The polypeptide or protein may comprise a complete protein or a functional variant thereof.

In some embodiments, the polypeptide and/or protein expressed by the nucleic acid encoding the polypeptide and/or protein of interest comprises or is one or more of (a) an antigen; (b) a therapeutic protein or polypeptide, a fragment thereof, a fragment or variant thereof; and (c) other polypeptides or proteins.

The PEG lipid represented by formula (I) or lipid nanoparticles (LNPs) containing the PEG lipid represented by formula (I) avoid an immune reaction against the LNPs in a subject.

In some embodiments, repeated administrations (e.g., 2, 3, 4 or more administrations) of the PEG lipid represented by formula (I) or LNPs containing the PEG lipid represented by formula (I) do not induce an immune reaction against the LNPs in a subject.

In some embodiments, repeated administrations (e.g., 2, 3, 4 or more administrations) of the PEG lipid represented by formula (I) or LNPs containing the PEG lipid represented by formula (I) do not induce the production of IgM or IgG that binds to the LNPs. Not inducing the production of IgM or IgG that binds to the LNPs means inducing no natural IgM or IgG that binds to the LNPs, or inducing a low level of IgM or IgG which is insufficient to cause ABC.

The PEG lipid represented by formula (I) or LNPs containing the PEG lipid represented by formula (I) reduce an immune reaction against the LNPs in a subject.

In some embodiments, repeated administrations (e.g., 2, 3, 4 or more administrations) of LNPs containing the PEG lipid represented by formula (I) induce less IgM or IgG, for example, a reduction by at least 30%, 35%, 40%, 45%, 50%, 55%, 60%, 65%, 70%, 75%, 80%, 85%, 90%, 91%, 92%, 93%, 94%, 95%, 96%, 97%, 98%, 99% or 100%, compared to control LNPs (e.g., LNPs whose PEG lipid is DMG-PEG, wherein DMG refers to 1,2-dimyristoyl-sn-glycerol, and DMG-PEG is a polyethylene glycol derivative of DMG).

The PEG lipid represented by formula (I) or LNPs containing the PEG lipid represented by formula (I) avoid the occurrence of accelerated blood clearance (ABC).

In some embodiments, repeated administrations of the PEG lipid represented by formula (I) or LNPs containing the PEG lipid represented by formula (I) also do not cause the occurrence of accelerated blood clearance (ABC).

The PEG lipid represented by formula (I) or LNPs containing the PEG lipid represented by formula (I) reduce the degree of accelerated blood clearance (ABC).

In some embodiments, compared to control LNPs (e.g., LNPs whose PEG lipid is DMG-PEG), when using LNPs containing the PEG lipid represented by formula (I) to deliver an active ingredient for a second time or more times, the clearance rate of the active ingredient is less, for example, reduced by at least 1%, 5%, 10%, 20%, 25%, 30%, 35%, 40%, 45%, 50%, 55%, 60%, 65%, 70%, 75%, 80%, 85%, 90%, 95%, 98% or 100%.

In some embodiments, compared to control LNPs (e.g., LNPs whose PEG lipid is DMG-PEG), when using LNPs containing the PEG lipid represented by formula (I) to deliver an active ingredient for a second time or more times, the clearance rate of the active ingredient is less, for example, reduced by at least 1%~5%, 10%~100%, 10%~50%, 20%~100%, 20%~50%, 30%~100%, 30%~50%, 40%~100%, 40%~80%, 50%~90% or 50%~100%.

Additionally, the present disclosure also provides a composition comprising the lipid nanoparticle according to any of the preceding embodiments, and a biologically acceptable carrier. As used herein, the term "biologically acceptable carrier" refers to those carriers that have no significant irritating effect on natural or artificial organisms (including humans, animals, plants, or *in vivo* or *in vitro* tissues or cells thereof; single-cell organisms such as bacteria and fungi; and artificial cells) and do not impair the activity and performance of the active ingredient in the lipid nanoparticle.

Additionally, the present disclosure also provides a pharmaceutical composition comprising the lipid nanoparticle according to any of the preceding embodiments, and a pharmaceutically acceptable carrier.

The pharmaceutical composition described above comprises the lipid nanoparticle described above, and has corresponding advantages thereof.

Furthermore, the present disclosure also provides a use of the lipid nanoparticle according to any of the preceding embodiments or the pharmaceutical composition according to any of the preceding embodiments in the manufacture of a medicament.

In some embodiments, the medicament is for use in the treatment and/or prevention of a disease.

In some embodiments, the medicament is for use in gene therapy, genetic vaccination, protein replacement therapy, antisense therapy, or therapy via interfering RNA.

In some embodiments, the lipid particle is used as a vehicle for transferring or delivering an active ingredient. In some embodiments, the active ingredient is a pharmaceutically active ingredient, for example, a nucleic acid.

In some embodiments, the medicament is for use in treating a disease related to the polypeptide or protein of interest as described above.

In some embodiments, the medicament is for use in the treatment and/or prevention of one or more of rare diseases, cancers, infectious diseases, autoimmune diseases, metabolic diseases, neurological diseases, cardiovascular diseases, transplant rejection, inflammatory reactions, genetic diseases, and musculoskeletal diseases.

In some embodiments, the rare diseases include one or more of osteogenesis imperfecta (Brittle Bone Disease), Wilson's disease, spinal muscular atrophy (SMA), Huntington's disease, Rett syndrome, amyotrophic lateral sclerosis (ALS), Duchenne type muscular dystrophy, Friedrich's ataxia, methylmalonic acidemia (MMA), cystic fibrosis (CF), glycogen storage disease 1a (GSD1a), glycogen storage disease III (GSD III), Crigler-Najjar syndrome, ornithine transcarbamylase deficiency (OTCD), propionic acidemia (PA), phenylketonuria (PKU), hemophilia A, hemophilia B, β-thalassemia, Lafora disease, Dravet syndrome (DS), Alexander disease, Leber's congenital amaurosis (LCA), myelodysplastic syndrome (MDS), and homocystinuria due to CBS deficiency. Additionally, rare diseases on www.orpha.net/consor/cgi-bin/Disease_Search_List.php and rarediseases.info.nih.gov/diseases are also incorporated herein by reference.

In some embodiments, the cancers include one or more of hematological malignancies, lung cancer, liver cancer, kidney cancer, head and neck cancer, esophageal cancer, gastric cancer, colorectal cancer, pancreatic cancer, brain cancer, prostate cancer, gallbladder cancer, ovarian cancer, breast cancer, cervical cancer, endometrial cancer, bladder cancer and melanoma.

In some embodiments, the infectious diseases include diseases caused by infection with one or more of viruses, fungi and bacteria.

In some embodiments, the autoimmune diseases include one or more of acute idiopathic thrombocytopenic purpura, chronic idiopathic thrombocytopenic purpura, systemic lupus erythematosus, rheumatoid arthritis, psoriasis, inflammatory bowel disease, multiple sclerosis, celiac disease, type 1 diabetes and diffuse toxic goiter.

In some embodiments, the genetic diseases include one or more of hemophilia, thalassemia and Gaucher's disease.

In some embodiments, the neurological diseases include one or more of amyotrophic lateral sclerosis, Alzheimer's disease and glioma.

In some embodiments, the medicament is a vaccine.

In some embodiments, the medicament is a nucleic acid drug, wherein the nucleic acid includes at least one of RNA and DNA. In one alternative specific example, the DNA is a plasmid. In one alternative specific example, the RNA is one or more of mRNA, antisense oligonucleotides, rRNA, miRNA, tRNA, siRNA, snRNA, shRNA, sgRNA and Cas9 mRNA.

Additionally, the present disclosure provides a use of the lipid nanoparticle according to any of the preceding embodiments in the preparation of a vehicle for transferring or delivering an active ingredient. In some embodiments, the active ingredient is a pharmaceutically active ingredient, for example, a nucleic acid.

Additionally, the present disclosure also provides a method for delivering the active ingredient described above to mammalian cells, the method comprising administering to a mammal the lipid nanoparticle according to any of the preceding embodiments containing the active ingredient, said administering comprising contacting the cells of the mammal with the lipid nanoparticle, thereby delivering the active ingredient to the cells of the mammal. In some embodiments, the active ingredient is a pharmaceutically active ingredient, for example, a nucleic acid.

In some embodiments, the nucleic acid includes at least one of RNA, messenger RNA (mRNA), antisense oligonucleotides, DNA, plasmids, ribosomal RNA (rRNA), microRNA (miRNA), transfer RNA (tRNA), small interfering RNA (siRNA), small nuclear RNA (snRNA), small hairpin RNA (shRNA), single-guide RNA (sgRNA) and Cas9 mRNA.

In other embodiments, the active ingredient is a transfection reagent or a detection reagent.

Furthermore, the present disclosure also provides a medicament comprising the PEG lipid according to any of the preceding embodiments, the lipid nanoparticle according to any of the preceding embodiments, or the pharmaceutical composition according to any of the preceding embodiments.

In some embodiments, the disease or disorder that can be prevented or treated with the medicament is as described above.

Additionally, the present disclosure also provides a medicament comprising the pharmaceutical composition according to any of the preceding embodiments.

In some embodiments, the disease or disorder that can be prevented or treated with the medicament is as described above.

Furthermore, the present disclosure provides a method for preventing or treating a disease or disorder, comprising administering the lipid nanoparticle, pharmaceutical composition or medicament of the present invention to a subject in need thereof.

In some embodiments, the disease or disorder to be prevented or treated is as described above.

The method for preventing or treating the disease as described above has corresponding advantages due to the use of the lipid nanoparticle, pharmaceutical composition or medicament as described above.

Additionally, the present disclosure also provides a method for reducing accelerated blood clearance (ABC) in a subject undergoing a treatment, the method comprising delivering a pharmaceutically active ingredient with lipid nanoparticles (LNPs) containing the PEG lipid according to any of the preceding embodiments.

Additionally, the present disclosure also provides a method for delivering lipid nanoparticles (LNPs) encapsulating a pharmaceutically active ingredient to a subject without the influence of an immune reaction promoting accelerated blood clearance (ABC) against subsequent doses of the LNPs, the method comprising:
providing the LNPs encapsulating the pharmaceutically active ingredient, which comprise the PEG lipid according to any of the preceding embodiments;
administering a first dose of the LNPs to the subject, wherein the first dose of the LNPs does not induce an immune response promoting the ABC when a second dose of the LNPs is administered; and
administering the second dose of the LNPs to the subject, wherein the subject does not have an ABC reaction to the second dose of the LNPs.

### Examples

To make the objectives and technical solutions of the present disclosure clearer, detailed descriptions are provided below in conjunction with specific examples. Obviously, the described examples are only part of the embodiments of the present disclosure, and not all of them. Based on the embodiments in the present disclosure, all other embodiments obtained by a person of ordinary skill in the art without creative effort shall fall within the protection scope of the present disclosure. Unless particularly specified, reagents and instruments used in the examples are conventional choices in the art. Experimental methods not specified with specific conditions in the examples are carried out under conventional conditions, for example, conditions described in literature, books, or achieved by methods recommended by the manufacturer.

In the examples, "Hep" refers to n-heptane, "EA" refers to ethyl acetate, "DMF" refers to N,N-dimethylformamide, "TEA" refers to triethylamine, "EDCI" refers to 1-(3-dimethylaminopropyl)-3-ethylcarbodiimide hydrochloride, "DMAP" refers to 4-dimethylaminopyridine, and "DCM" refers to dichloromethane.

### Example 1. Synthesis Process of Compound 001

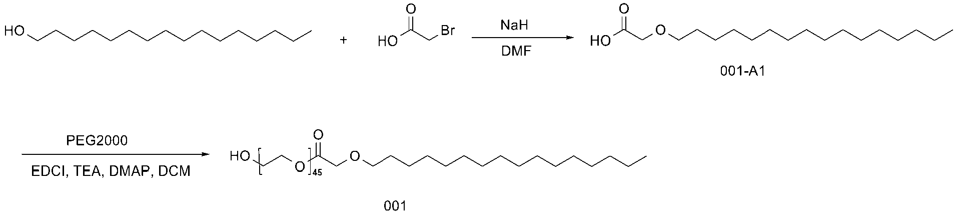

### Step 1: Synthesis of Compound 001-A1

### Experimental Procedure

To a 250 mL single-neck flask was added 1-hexadecanol (10.0 g, 41.25 mmol), dissolved in DMF (100 mL), cooled to below 10°C, and slowly added with NaH (4.95 g, 123.75 mmol) in portions and stirred for 30 min while maintaining the temperature after the addition. Bromoacetic acid (6.88 g, 49.5 mmol) was added slowly to the reaction, reacted for 30 min while continuing the maintenance of the temperature after addition, then transferred to room temperature (25°C) and reacted for 15 h. The reaction solution was added with water (200 mL), adjusted to pH 6.5~7.5 with dilute hydrochloric acid, then extracted twice with EA (200 mL). The EA phases were combined. The combined EA phases were then washed with saturated brine (200 mL). The EA phase was collected and concentrated under reduced pressure. The concentrate was subjected to silica gel column chromatography, eluted with Hep:EA=3: 1, to give compound 001-A1 a white solid (4.5 g, a yield of 36.3%).

### Step 2: Synthesis of Compound 001

### Experimental Procedure

To a 250 mL single-neck flask was added PEG2000 (19.96 g, 9.98 mmol), DCM (100 mL) was added and sonicated to dissolve. Then EDCI (1.91 g, 9.98 mmol), TEA (1.05 g, 9.98 mmol), and DMAP (0.24 g, 2.0 mmol) were added, stirred for 5 min, then compound 001-A1 (2 g, 6.66 mmol) was added to react at room temperature for 15 h. Water (200 mL) was added to the reaction and stirred for 10 min, the lower organic phase was separated and collected, and the aqueous phase was further extracted once with DCM (100 mL). The DCM phases were combined, then washed once with saturated brine (100 mL), and finally the DCM phase was concentrated under reduced pressure. The concentrate was purified by silica gel column chromatography, eluted with Hep:EA=15:1. The obtained product was further purified by reverse-phase column chromatography, gradiently eluted with EtOH:H₂O (20%~70%), to give compound 001 (800 mg) with a purity of 99.3% and a yield of 5.3%.

¹H NMR (400 MHz, CDCl₃) δ 4.31 (m, 2H), 4.10 (s, 2H), 3.76-3.57 (m, 184H), 3.52 (t, *J* = 6.7 Hz, 2H), 1.67-1.55 (m, 2H), 1.40-1.20 (m, 26H), 0.88 (t, *J* = 6.8 Hz, 3H).

LC-MS (ESI): Mn=2277.

### Example 2 Synthesis Process of Compound 003

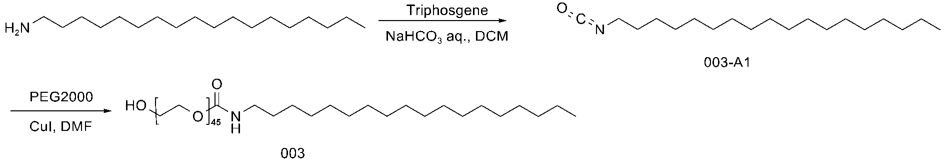

### Step 1: Synthesis of Compound 003-A1

### Experimental Procedure

To a 250 mL single-neck flask was added 1-octadecylamine (5 g, 18.55 mmol), dissolved in DCM (100 mL), then added with saturated aqueous sodium bicarbonate solution (50 mL) and cooled to below 10°C. Triphosgene (2.2 g, 7.42 mmol) was added to the reaction, and reacted for 1 h. The organic phase was separated and collected, and the aqueous phase was further extracted once with DCM (100 mL). The DCM phases were combined and washed once with saturated brine (100 mL). The DCM phase was concentrated under reduced pressure. The concentrate was subjected to silica gel column chromatography, eluted with Hep:EA=5:1, to give compound 003-A1 as a solid (4.64 g, a yield of 84.6%).

### Step 2: Synthesis of Compound 003

### Experimental Procedure

To a 250 mL single-neck flask was added PEG2000 (30.46 g, 15.23 mmol), dissolved in DMF (60 mL), then added with cuprous iodide (0.39 g, 2.03 mmol), finally added with compound 003-A1 (3 g, 10.15 mmol), and reacted at room temperature for 3 h. The solid was removed by filtration, and the filtrate was added with water (200 mL) and extracted twice with EA (100 mL). Ammonia water was added dropwise with stirring to remove copper ions until the EA phase became colorless. The EA phases were combined, further washed once with saturated brine (100 mL), and the EA phase was concentrated under reduced pressure. The concentrate was subjected to silica gel column chromatography, eluted with Hep:EA=5:1. The obtained product was further purified by reverse-phase column chromatography, gradiently eluted with EtOH:H₂O (20%~70%), to give compound 003 (1.4 g) with a purity of 98.9% and a yield of 6.2%.

¹H NMR (400 MHz, CDCl₃) δ 4.25-4.16 (m, 2H), 3.86-3.78 (m, 1H), 3.77-3.53 (m, 184H), 3.15 (dd, *J =* 13.4, 6.8 Hz, 2H), 1.47 (m, 2H), 1.25 (m, 30H), 0.88 (t, *J* = 6.8 Hz, 3H).

LC-MS (ESI): Mn=2230.

### Example 3: Synthesis Process of Compound 006

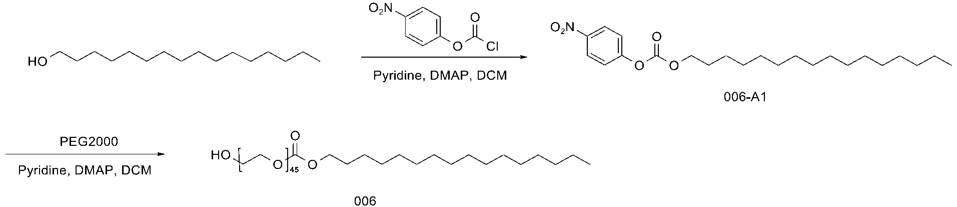

### Step 1: Synthesis of Compound 006-A1

### Experimental Procedure

To a 100 mL single-neck flask was added 1-hexadecanol (5 g, 20.62 mmol), dissolved in DCM (50 mL), then added with pyridine (2.45 g, 30.39 mmol) and DMAP (0.76 g, 6.19 mmol), stirred at room temperature, further slowly added with p-nitrophenyl chloroformate (4.99 g, 24.74 mmol) in portions, and stirred at room temperature for 3 h. Water (100 mL) was added and stirred, and the organic phase was separated and collected, and the aqueous phase was further extracted with DCM (100 mL). The DCM phases were combined, washed with saturated brine (100 mL), and concentrated under reduced pressure. The concentrate was subjected to silica gel column chromatography, eluted with Hep:EA=20:1, to give compound 006-A1 as a solid (8.1 g, a yield of 96.9%).

### Step 2: Synthesis of Compound 006

### Experimental Procedure

To a 500 mL single-neck flask was added PEG2000 (49.16 g, 24.58 mmol), dissolved in DCM (150 mL), then added with pyridine (4.9 g, 61.45 mmol) and DMAP (0.45 g, 3.69 mmol), finally added with compound 006-A1 (5 g, 12.29 mmol), and reacted at room temperature for 3 days. Water (200 mL) was added to the reaction solution and stirred, the organic phase was separated and collected, and the aqueous phase was extracted with DCM (100 mL). The organic phases were combined, washed once with saturated brine (100 mL), the organic phase was collected and concentrated under reduced pressure. The concentrate was subjected to silica gel column chromatography, eluted with Hep:EA=15:1. The obtained product was further purified by reverse-phase column chromatography, gradiently eluted with EtOH:H₂O (20%~70%), to give compound 006 (9.3 g) with a purity of 99.1% and a yield of 31.7%.

¹H NMR (600 MHz, CDCl₃) δ 4.27-4.23 (t, *J* = 4.8 Hz, 2H), 4.13-4.06 (t, *J* = 6.8 Hz, 2H), 3.62 (m, 184H), 1.66-1.59 (m, 2H), 1.36-1.29 (m, 2H), 1.29-1.19 (m, 24H), 0.85 (t, *J=* 6.8 Hz, 3H).

LC-MS (ESI): Mn=2156.

### Example 4: Synthesis of Compound 002

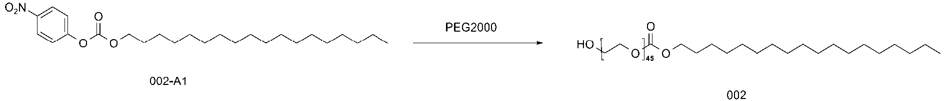

Compound 002-A1 was synthesized with reference to Step 1 of Example 3, then compound 002-A1 was used to synthesize compound 002 with reference to Step 2 of Example 3, with compound 002-A1 (2 g, 4.59 mmol) and PEG2000 (13.8 g, 6.89 mmol), affording compound 002 (1.67 g) with a purity of 98.3% and a yield of 15.6%.

¹H NMR (400 MHz, CDCl₃) δ 4.27 (m, 2H), 4.12 (t, *J* = 6.8 Hz, 2H), 3.65 (d, *J* = 7.7 Hz, 184H), 1.70 - 1.59 (m, 2H), 1.38-1.21 (m, 30H), 0.88 (t, *J =* 6.8 Hz, 3H).

LC-MS (ESI): Mn=2240

### Example 5 Synthesis of Compound 004

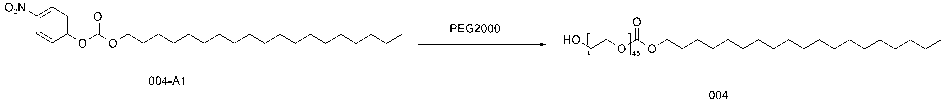

Compound 004-A1 was synthesized with reference to Step 1 of Example 3, then compound 004-A1 was used to synthesize compound 004 with reference to Step 2 of Example 3, with compound 004-A1 (1.5 g, 3.34 mmol) and PEG2000 (10.0 g, 5.00 mmol), affording compound 004 (2.3 g) with a purity of 98.8% and a yield of 30.14%.

¹H NMR (400 MHz, CDCl₃) δ 4.31-4.24 (m, 2H), 4.12 (t, *J =* 6.8 Hz, 2H), 3.65 (m, 184H), 1.67-1.60 (m, 2H), 1.25 (m, 32H), 0.88 (t, *J=* 6.8 Hz, 3H).

LC-MS (ESI): Mn=2287.

### Example 6: Synthesis of Compound 005

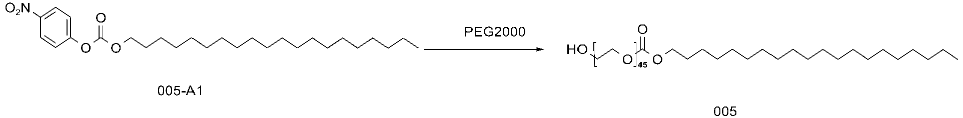

Compound 005-A1 was synthesized with reference to Step 1 of Example 3, then compound 005-A1 was used to synthesize compound 005 with reference to Step 2 of Example 3, with compound 005-A1 (1.2 g, 2.59 mmol) and PEG2000 (7.76 g, 3.88 mmol), affording compound 005 (2.12 g) with a purity of 98.1% and a yield of 36.49%.

¹H NMR (400 MHz, CDCl₃) δ 4.27 (m, 2H), 4.12 (t, *J* = 6.8 Hz, 2H), 3.65 (m, 184H), 1.63 (m, 2H), 1.28 (m, 34H), 0.88 (t, *J* = 6.8 Hz, 3H).

LC-MS (ESI): Mn=2243.

### Example 7: Synthesis of Compound 013

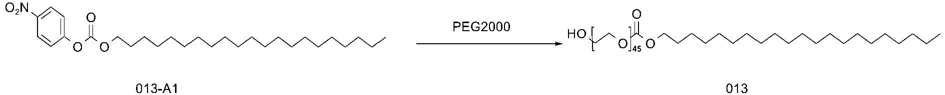

Compound 013-A1 was synthesized with reference to Step 1 of Example 3, then compound 013-A1 was used to synthesize compound 013 with reference to Step 2 of Example 3, with compound 013-A1 (1.6 g, 3.35 mmol) and PEG2000 (10.05 g, 5.02 mmol), affording compound 013 (2.9 g) with a purity of 98.5% and a yield of 39.7%.

¹H NMR (400 MHz, CDCl₃) δ 4.314.25 (m, 2H), 4.12 (t, *J* = 6.8 Hz, 2H), 3.65 (m, 184H), 1.63 (m, 2H), 1.25 (m, 36H), 0.88 (t, *J* = 6.8 Hz, 3H).

LC-MS (ESI): Mn=2180.

### Example 8: Synthesis of Compound 014

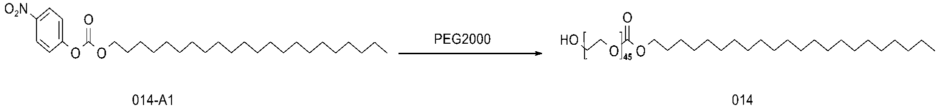

Compound 014-A1 was synthesized with reference to Step 1 of Example 3, then compound 014-A1 was used to synthesize compound 014 with reference to Step 2 of Example 3, with compound 014-A1 (2 g, 4.07 mmol) and PEG2000 (12.2 g, 6.11 mmol), affording compound 014 (2.58 g) with a purity of 99.2% and a yield of 29.5%.

¹H NMR (600 MHz, CDCl₃) δ 4.26-4.19 (m, 2H), 4.15-4.09 (t, *J =* 6.8 Hz, 2H), 3.77-3.42 (m, 184H), 1.65-1.55 (m, 2H), 1.34-1.15 (m, 38H), 0.83 (t, *J* = 6.8 Hz, 3H).

LC-MS (ESI): Mn=2150.

### Example 9: Synthesis of Compound 015

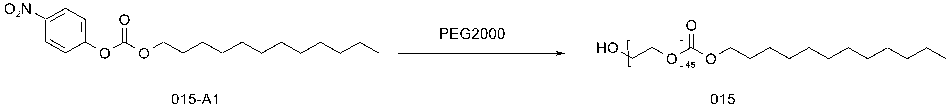

Compound 015-A1 was synthesized with reference to Step 1 of Example 3, then compound 015-A1 was used to synthesize compound 015 with reference to Step 2 of Example 3, with compound 015-A1 (2.1 g, 5.98 mmol) andPEG2000 (17.94 g, 8.96 mmol), affording compound 015 (2.68 g) with a purity of 98.2% and a yield of 20.1%.

¹H NMR (600 MHz, CDCl₃) δ 4.33-4.26 (m, 2H), 4.14 (t, *J* = 6.8 Hz, 2H), 3.87-3.58 (m, 184H), 1.72-1.62 (m, 2H), 1.41-1.24 (m, 18H), 0.90 (t, *J* = 6.8 Hz, 3H).

LC-MS (ESI): Mn=2230.

### Example 10: Synthesis of Compound 016

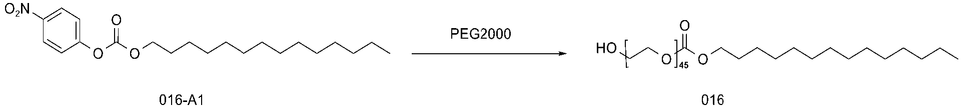

Compound 016-A1 was synthesized with reference to Step 1 of Example 3, then compound 016-A1 was used to synthesize compound 016 with reference to Step 2 of Example 3, with compound 016-A1 (2.3 g, 6.06 mmol) and PEG2000 (18.2 g, 9.09 mmol), affording compound 016 (3.0 g) with a purity of 98.9% and a yield of 22.1%.

¹H NMR (600 MHz, CDCl₃) δ 4.31-4.26 (m, 2H), 4.14 (t, *J* = 6.8 Hz, 2H), 3.76-3.57 (m, 184H), 1.72-1.64 (m, 2H), 1.41-1.24 (m, 22H), 0.90 (t, *J* = 6.8 Hz, 3H).

LC-MS (ESI): Mn=2240.

### Example 11 Synthesis of Compound 007

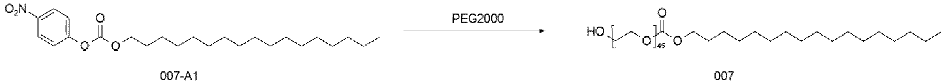

Compound 007-A1 was synthesized with reference to Step 1 of Example 3, then compound 007-A1 was used to synthesize compound 007 with reference to Step 2 of Example 3, with compound 007-A1 (1.5 g, 3.56 mmol) and PEG2000 (28.48 g, 14.24 mmol), affording compound 007 (2.61 g) with a purity of 99.1% and a yield of 33.2%.

¹H NMR (600 MHz, CDCl₃) δ 4.29-4.23 (t, *J* = 4.8 Hz, 2H), 4.13-4.07 (t, *J* = 6.8 Hz, 2H), 3.62 (m, 184H), 1.65-1.59 (m, 2H), 1.35-1.29 (m, 2H), 1.29-1.19 (m, 28H), 0.87 (t, *J=* 6.8 Hz, 3H).

LC-MS (ESI): Mn=2211.

### Experimental Example 1. Preparation of Lipid Nanoparticles Encapsulating Human Erythropoietin mRNA (hEPO mRNA)

(1) Formulating:
   A specified amount of hEPO mRNA stock solution, 0.2 M sodium acetate buffer, and DEPC water were added to a container, and mixed well to obtain a water phase;
   The cationic lipid (XI-1), a helper lipid (DSPC), a structural lipid (cholesterol), and a PEG lipid of the present disclosure or a control PEG lipid (DMG-PEG2000) were separately dissolved in absolute ethanol to prepare respective solutions at concentrations of 20 mg/mL, 10 mg/mL, 20 mg/mL, and 25 mg/mL, respectively. The above four solutions were pipetted at a molar ratio of cationic lipid (XI-1): DSPC: CHO-HP: PEG lipid of 48:10:40.5:1.5, and mixed well to prepare an alcohol phase.
(2) Encapsulation: The water phase and the alcohol phase were aspirate into water phase and alcoholic phase syringes at a flow rate of 9 mL/min: 3 mL/min by using a microfluidic preparation instrument (MPE-L2), and encapsulation was carried out at a flow rate of water phase: alcohol phase = 12 mL/min: 4 mL/min to obtain mRNA-encapsulating lipid nanoparticle (mRNA-LNP).
(3) Dialysis: The product of step (2) was loaded in a dialysis bag and placed in a Tris Buffer-8% (m/V) sucrose solution for replacement to remove ingredients such as residual ethanol, unassembled lipids. Dialysis was conducted for 2 h with magnetic stirring at room temperature and under protection from light (dialysate was replaced every 1 hour).
(4) The product of step (3) was sterilized by passing through a 0.22 µm microporous membrane, and then packaged.

A variety of lipid nanoparticle formulations (LNP formulations) encapsulating hEPO mRNA were prepared, wherein the PEG lipids contained in the variety of LNP formulations were different from each other, and each LNP formulation had a hEPO mRNA concentration of 0.2 µg/µL, a molar ratio of hEPO mRNA to Lipid (cationic lipid (XI-1)) of 1:4, a particle size of 80 nm-130 nm, and an encapsulation efficiency of 85% or higher.

### Experimental Example 2. Assay of hEPO Concentration in Serum of Rats after Multiple Administrations

Animal preparation: SD rats of 5-7 weeks old, half male and half female, were selected and raised in an SPF grade breeding room. Animal testing was conducted in strict accordance with the guidelines of national health institutions and animal ethics requirements.

*In vivo* Delivery: prior to injection of the test LNP formulation, the LNP formulation was gently and repeatedly inverted to thoroughly mix the sample of the formulation. A corresponding amount of the LNP formulation was aspirated with a 2 ml syringe, and the LNP formulation was injected by tail vein injection (IV), with 4~6 rats (half male and half female) in duplicate per formulation. Each rat was injected with the corresponding human Erythropoietin mRNA (hEPO mRNA)-encapsulated LNP formulations prepared in Experimental Example 1 at a dose of 0.5 mg/kg mRNA with one administration per week for 4~6 consecutive doses.

At 3h/8h/24h after the first injection of the LNP formulations, and at 8h after the second, third, fourth, fifth, and sixth injections of the LNP formulations, whole blood was collected from the rats, and serum was obtained. The procedures for obtaining serum included: collecting blood samples from the rats and placing them in tubes without an anticoagulant agent; allowing natural coagulation at room temperature for 30min~60min, centrifuging at 3500 rpm for 10 min after blood coagulation to obtain the supernatant as serum.

The particular method for determining the hEPO concentration in serum is as follows:

### 1. Solution Preparation

**0.05% PBST wash solution:** 250 mL of 20×PBS was taken and added with water to a set volume of 5 L, added with 2.5 mL Tween20 and mixed well, set aside; **3% BSA blocking solution:** 3.0 g BSA powder was weighed out by an electronic balance, added with the 0.05% PBST to a set volume of 100 mL and mixed well, set aside; **1% BSA dilution solution:** 1.0 g BSA powder was weighed out by an electronic balance, added with the 0.05% PBST to a set volume of 100 mL and mixed well, set aside; **Reagents for standard curve:** 1) Dilution of stock solution: 2 µL of a stock solution (Cat#: EPO-H4214, Manufacturer: Acro) was taken (initial concentration: 0.6 mg/mL), added to 998 µL of a dilution solution and mixed well, and at this time the concentration of the standard was 1.2 µg/mL; 2) dilution for standard curve: 3-fold serial dilution.

### 2. ELISA Assay:

(1) Coating: the coating antibody (Cat#: ab272358, Manufacturer: Abcam) was diluted with PBS to a concentration of 1 µg/mL, at 100 µL/well, coating at 4°C overnight; (2) Plate washing: the plate was washed using the 0.05% PBST wash solution, at 250 µL/well, washed 3 times; (3) Blocking: 3% BSA prepared with the 0.05% PBST wash solution, at 250 µL/well, incubated at 37°C for 1 h; (4) Plate washing: the plate was washed using the 0.05% PBST wash solution, at 250 µL/well, washed 3 times; (5) Incubation of samples to be determined: the diluted samples for standard curve and samples to be determined were added according to plate layout, at 100 µL/well, incubated at 37°C for 2h; (6) Plate washing: the plate was washed using the 0.05% PBST wash solution, at 250 µL/well, washed 3 times; (7) Incubation of dtector antibody: the detection antibody (biotin-labeled) was prepared at 0.2 µg/mL using the 1% BSA dilution solution, at 100 µL/well, incubated at 37°C for 1h; (Cat#: ab272359, Manufacturer: Abcam); (8) Plate washing: the plate was washed using the 0.05% PBST wash solution, at 250 µL/well, washed 3 times; (9) SA-HRP incubation: A secondary detection antibody (Cat#: 21126 Manufacturer: Invitrogen) was diluted at 1:5000 using the dilution solution, at 100 µL/well, incubated at 37°C for 45min; (10) Plate washing: the plate was washed using the 0.05% PBST wash solution, at 250 µL/well, washed 3 times; (11) Color development: TMB (Cat#: PR1200, Manufacturer: Solarbio) was added for color development, at 100 µL/well, incubated at 37°C for 5 min; (12) Stop: a stop solution was added at 50 µL/well; (13) Plate reading: OD450 and OD630 on an ELISA reader; (14) Data analysis: OD450-OD630.

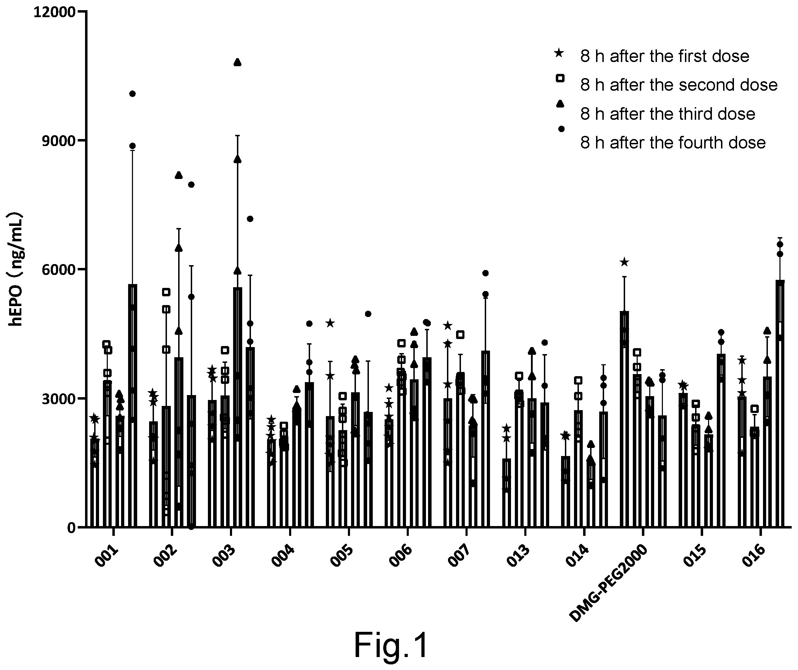

The assay results of the hEPO concentration in serum are shown in Figure 1. As can be seen from Figure 1, when multiply dosed, the serum hEPO concentration in rats dosed the LNP formulation containing DMG-PEG2000 gradually decreased as the number of doses increased, suggesting that the LNP formulation containing DMG-PEG2000 exhibited the accelerated blood clearance (ABC) phenomenon. When multiply dosed, the serum hEPO concentration in rats dosed the LNP formulations containing the PEG lipids of the present disclosure did not exhibit a gradual decrease as the number of doses increased, indicating that the LNP formulations containing the PEG lipids of the present disclosure can significantly alleviate the accelerated blood clearance (ABC) phenomenon.

### Experimental Example 3. Assay of Anti-PEG IgG in Serum of Rats after Multiple Administrations

Serum acquisition: from rats in Experimental Example 2, whole blood was collected at 96h or 120h after each dose, and serum was obtained. The method for obtaining serum was as shown in Experimental Example 2.

The specific method for the assay of serum anti-PEG IgG is as follows:
1. Solution Preparation
   0.05% CHAPS wash solution: 200 mL of 20×PBS was taken and added with water to a set volume of 4 L, added with 2 g CHAPS and mixed well, and set aside;
2. 3% BSA blocking solution: 9.0 g BSA powder was weighed out, added with 0.05% PBS-CHAPS to a set volume of 300 mL and mixed well, set aside;
3. 1% BSA dilution solution: 1.0 g BSA powder was weighed out, added with 0.05% PBS-CHAPS to a set volume of 100 mL and mixed well, set aside;

### 2. ELISA Assay

(1) Coating reagent: the coating reagent (coating the corresponding PEG) was diluted with 0.1 M carbonate-bicarbonate solution (pH=9.51) to 10 µg/mL, at 100 µL/well, overnight at 4°C; (2) Plate washing: the plate was washed using the 0.05% CHAPS wash solution (Cat#: ST1146-5g, Manufacturer: Beyotime), at 250 µL/well, washed 3 times; (3) Blocking: 3% BSA prepared with the 0.05% CHAPS wash solution, at 250 µL/well, incubated at 37°C for 1.5h; (4) Plate washing: the plate was washed using the 0.05% CHAPS wash solution, at 250 µL/well, washed 3 times; (5) Incubation of samples to be determined: the diluted samples to be determined were added according to plate layout, at 100 µL/well, incubated at 37°C for 2h; (6) Plate washing: the plate was washed using the 0.05% CHAPS wash solution, at 250 µL/well, washed 3 times; (7) Incubation of Rat-HRP: A secondary detection antibody (Cat#: 62-9520, Manufacturer: Invitrogen) was diluted at 1:4000 with 1% BSA in the 0.05% CHAPS wash solution, at 100 µL/well, incubated at 37°C for 1h; (8) Plate washing: the plate was washed using the 0.05% PBST dilution solution, at 250 µL/well, washed 3 times; (9) Color development: TMB was added for color development, at 100 µL/well, incubated at 37°C for 10min; (10) Stop: a stop solution was added at 50 µL/well; (11) Plate reading: OD450 and OD630 on an ELISA reader; (12) Data analysis: OD450-OD630.

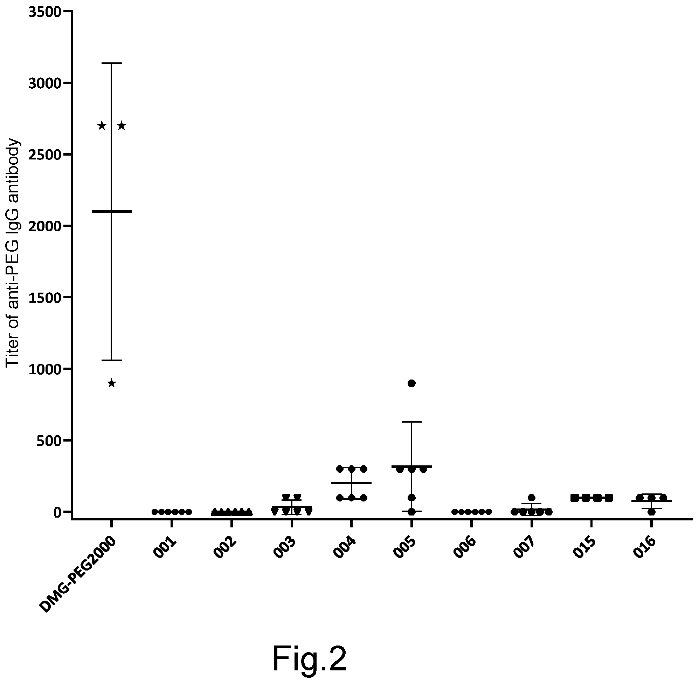

The assay results of the anti-PEG IgG in serum at 96h after the third dose are shown in Figure 2. As can be seen from Figure 2, compared to rats administered the LNP formulation containing DMG-PEG2000, rats administered LNP formulations containing the PEG lipid of the present disclosure had very low titers of the anti-PEG IgG antibodies in their serum. Especially for the LNP formulation containing 001, the LNP formulation containing 002, the LNP formulation containing 006, and the LNP formulation containing 007, the assay results of the anti-PEG IgG antibodies in rat serum were essentially negative, suggesting that the LNPs containing the PEG lipid of the present disclosure can result in significantly reduced or no production of the anti-PEG IgG antibodies.

### Experimental Example 4: Assay of Cytokines IL-6 and IFN-γ in Serum of Rats after Administration

Serum acquisition: from rats in Experimental Example 2, whole blood was collected at 5h after the first dose, and serum was obtained. The contents of IL-6 and IFN-γ in serum were detected using a kit (LEGENDplex^{™} Rat Inflammation Panel (13-plex) w/VbP V02 (BIOLEGEND, cat#741396)), particularly by procedures including:
1. Preparing 1× Wash Buffer, standards, and Beads according to the method provided in the kit.
2. Sample addition and incubation: standards and 4-fold diluted rat serum samples were added to a V-bottom Plate, then Beads were added to each well and the V-bottom Plate was shaken at 500 rpm on a microplate shaker, and incubated at room temperature under protection from light for 2h; after the incubation was complete, the plate was washed using 1× Wash Buffer.
3. Addition of the detection antibody: 25 µL Detection Antibody was added to each well of the V-bottom Plate, the V-bottom Plate was shaken at 500 rpm on a microplate shaker, and incubated at room temperature under protection from light for 1h.
4. After the incubation was complete, 25 µL SA-PE was directly added to each well, the V-bottom Plate was shaken at 500 rpm on a microplate shaker, and incubated at room temperature under protection from light for 30min.
5. After the incubation was complete, the plate was washed once, then added with 200 µL 1× Wash Buffer for re-suspending, and determined in a flow cytometry.

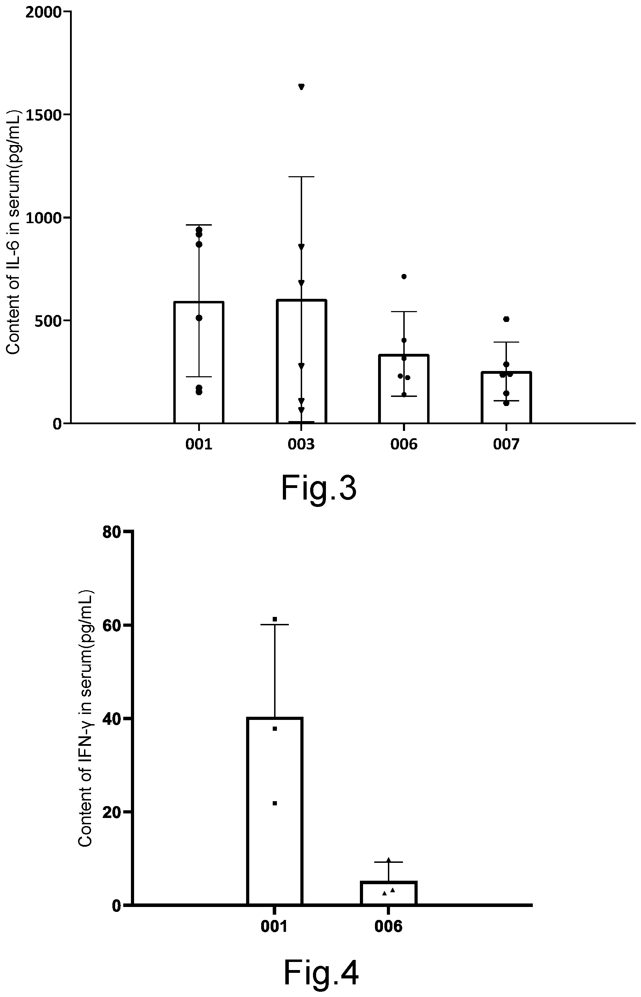

The results are shown in Figures 3 and 4. The cytokine responses induced by LNP formulations prepared with PEG lipids of different structures showed significant differences. When the linker connecting the polyethylene glycol part and the alkyl tail chain in the PEG lipid was different, the contents of IL-6 or IFN-γ induced by the LNP formulations, which were prepared with the corresponding PEG lipids, showed significant differences. For example, the content of IFN-γ induced by the LNP formulation, which was prepared with the PEG lipid indicated as 006, was significantly lower than that induced by the LNP formulation which was prepared with the PEG lipid indicated as 001.

## Claims

1. A PEG lipid represented by formula (I): 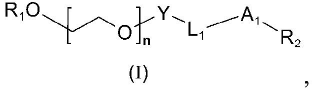 or a salt thereof, or a stereoisomer thereof,
wherein:
R₁ is H, HO-Z₁-Q₁- or H-Z₁-Q₁-,
Y is -C(=O)-, -C(=O)O-, -C(=O)NH-, -Q₂-Z₂-Q₃-, or a bond,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene or a bond,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl,
n is any integer from 30 to 60.

2. A PEG lipid represented by formula (IX): 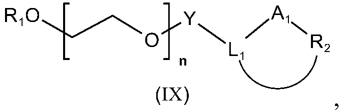 or a salt thereof, or a stereoisomer thereof,
wherein:
R₁ is H, HO-Z₁-Q₁- or H-Z₁-Q₁-,
Y is -C(=O)-, -C(=O)O-, -C(=O)NH-, -Q₂-Z₂-Q₃-, or a bond,
Z₁ and Z₂ are each independently -L₂-CH(OR₃)-L₃-, -L₂-C(=O)-L₃-, -L₂-CH(R₃)-L₃-, -L₂-, or a bond,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl,
n is any integer from 30 to 60,
L₁ together with A₁ and R₂ forms a four-, five- or six-membered ring.

3. A PEG lipid represented by formula (X): 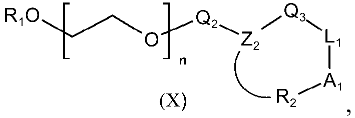 or a salt thereof, or a stereoisomer thereof,
wherein:
R₁ is H, HO-Z₁-Q₁- or H-Z₁-Q₁-,
Z₁ is -L₂-CH(OR₃)-L₃-, -L₂-C(=O)-L₃-, -L₂-CH(R₃)-L₃-, -L₂-, or a bond,
Z₂ is -L₂-CH(OR₃)-L₃-, -L₂-C(=O)-L₃-, -L₂-CH(R₃)-L₃-, or -L₂-,
Q₁ is -C(=O)-, -OC(=O)-, or a bond,
Q₂ is -C(=O)-, or a bond,
Q₃ is -C(=O)O-, -OC(=O)-, -O-, -S-, or a bond,
L₁ is C₁~C₂₀ hydrocarbylene or a bond,
A₁ is -C(=O)O-, -OC(=O)-, -OC(=O)O-, -O-, -S-, or a bond,
R₂ is C₄~C₂₄ hydrocarbyl,
the total number of carbon atoms in L₁ and R₂ is 10~35,
L₂ is C₁~C₄ hydrocarbylene,
L₃ is C₁~C₄ hydrocarbylene,
R₃ is H or C₁~C₃ hydrocarbyl,
n is any integer from 30 to 60,
Z₂ together with Q₃, L₁, A₁ and R₂ forms a four-, five- or six-membered ring.

4. The PEG lipid according to any one of claims 1 to 3, wherein:
R₁ is H, 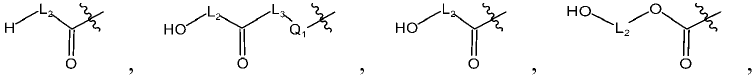 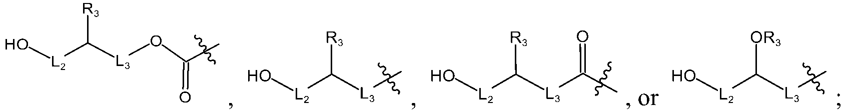
preferably, R₁ is H,

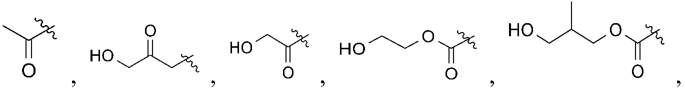

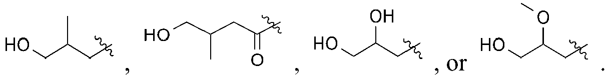

5. The PEG lipid according to claim 1 or 4, wherein the PEG lipid has a structure represented by formula (II): 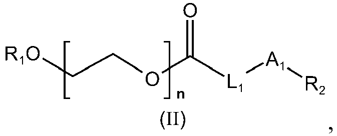 wherein R₁ is not H and is not an unsubstituted methyl.

6. The PEG lipid according to claim 5, wherein:
(1) R₁ is HO-Z₁-Q₁-, A₁ is a bond, and Z₁ and Q₁ are not simultaneously a bond;
preferably, Z₁ is -L₂-CH(OR₃)-L₃-, -L₂-C(=O)-L₃-, -L₂-CH(R₃)-L₃-, or -L₂-;
more preferably, Z₁ is -L₂-CH(OR₃)-L₃-, -L₂-C(=O)-L₃-, -L₂-CH(R₃)-L₃-, or -L₂-, and Q₁ is -C(=O)-, -OC(=O)-, or a bond,
wherein L₂ is preferably C₁~C₃ hydrocarbylene, L₃ is preferably C₁~C₃ hydrocarbylene, and R₃ is preferably H or C₁~C₃ alkyl;
preferably, R₁ is 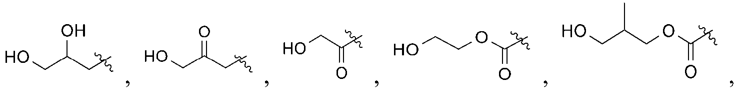 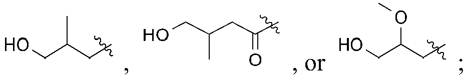 or
(2) R₁ is H-Z₁-Q₁-, wherein when Q₁ is a bond, Z₁ is not a bond and is not an unsubstituted methylene;
preferably, R₁ is H-Z₁-Q₁-, Z₁ is -L₂-, Q₁ is -C(=O)-, and A₁ is a bond, wherein L₂ is preferably C₁~C₃ hydrocarbylene, more preferably C₁~C₃ alkylene,
more preferably, R₁ is

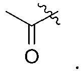

7. The PEG lipid according to claim 1, wherein the PEG lipid is represented by formula (II-1): 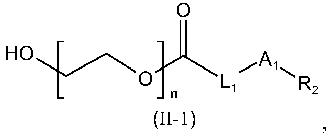
wherein A₁ is -OC(=O)-, -OC(=O)O-, -O- or -S-;
preferably, the PEG lipid is represented by formula (II-1-1):

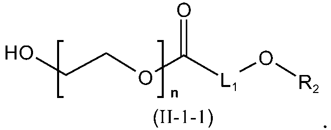

8. The PEG lipid according to claim 1 or 4, wherein the PEG lipid has a structure represented by formula (III), formula (IV), formula (V), formula (VI) or formula (VII):

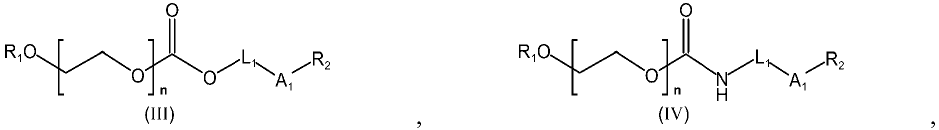

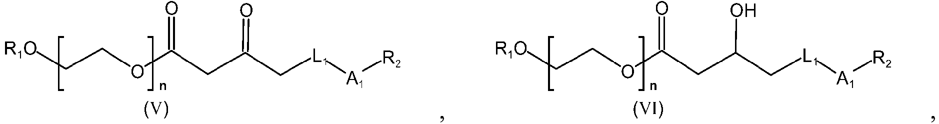

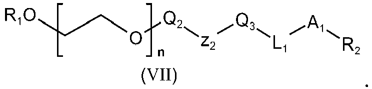

9. The PEG lipid according to any one of claims 1 and 4 to 8, wherein the PEG lipid has the following structure: 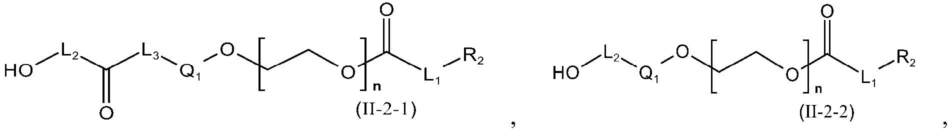 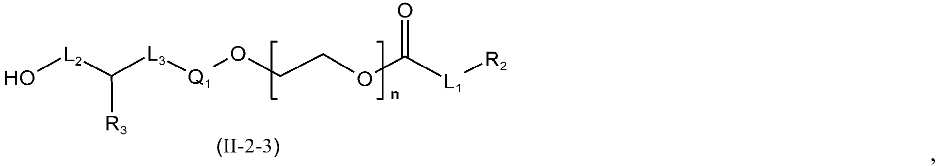 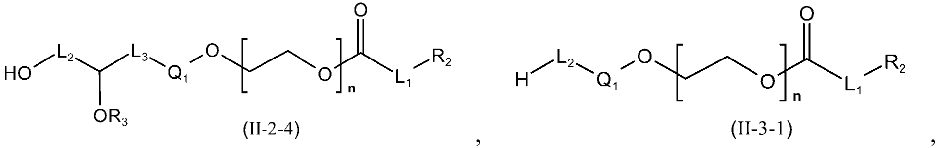 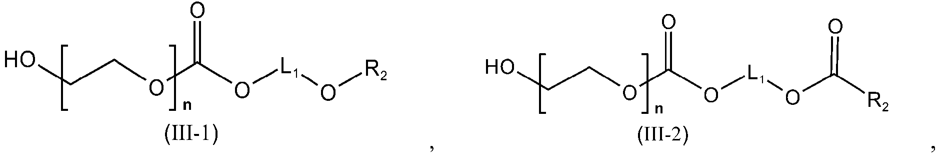 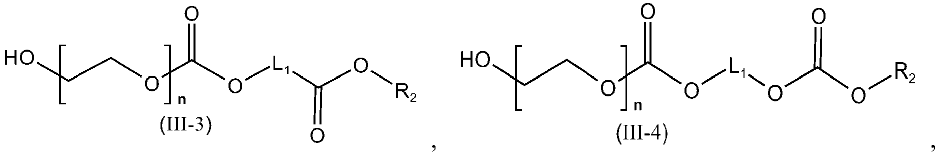 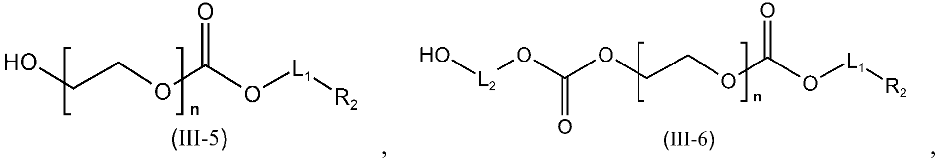 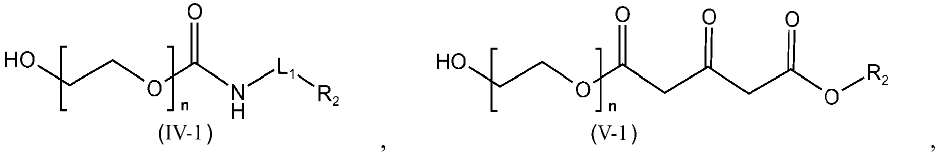 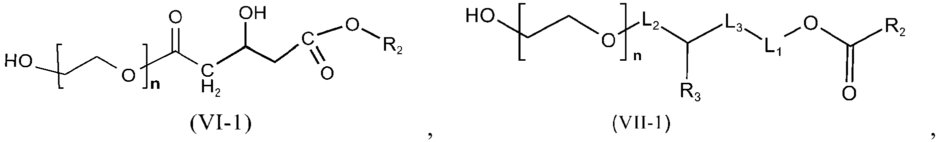 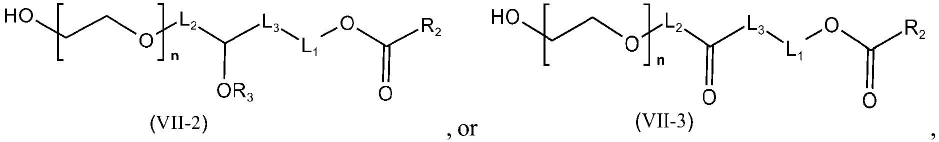 wherein in said formula (II-2-3), formula (II-2-4), formula (VII-1) or formula (VII-2), R₃ is preferably H or C₁~C₃ alkyl, more preferably H, C₁ alkyl or C₂ alkyl.

10. The PEG lipid according to claim 1 or 4, wherein the PEG lipid has the following structure:

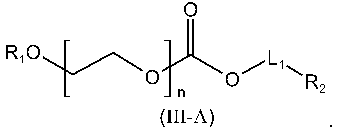

11. The PEG lipid according to claim 10, wherein the PEG lipid has the following structure:

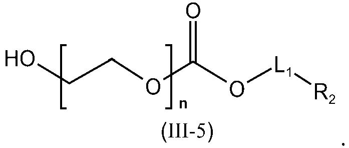

12. The PEG lipid according to any one of claims 1 to 11, wherein the total number of carbon atoms in L₁ and R₂ is 12~25.

13. The PEG lipid according to claim 12, wherein the total number of carbon atoms in L₁ and R₂ is 16~22.

14. The PEG lipid according to any one of claims 1 to 13, wherein L₁ is C_{1~}C₈ hydrocarbylene, or a bond.

15. The PEG lipid according to claim 14, wherein L₁ is C₁~C₈ alkylene, C₂~C₈ alkenylene or C₂~C₈ alkynylene.

16. The PEG lipid according to any one of claims 1 to 15, wherein R₂ is C₇~C₂₂ hydrocarbyl.

17. The PEG lipid according to claim 16, wherein R₂ is C₇~C₂₂ alkyl, C₇~C₂₂ alkenyl or C₇~C₂₂ alkynyl.

18. The PEG lipid according to any one of claims 1 to 17, wherein L₁ is straight C₁~C₈ alkylene, and R₂ is straight C₇~C₂₂ alkyl.

19. The PEG lipid according to any one of claims 10 to 18, wherein -L₁-R₂ is C₁₆~C₂₂ alkyl.

20. The PEG lipid according to claim 19, wherein -L₁-R₂ is straight C₁₆~C₂₂ alkyl.

21. The PEG lipid according to claim 19, wherein -L₁-R₂ is C₁₆~C₁₈ alkyl.

22. The PEG lipid according to claim 21, wherein -L₁-R₂ is straight C₁₆~C₁₈ alkyl.

23. The PEG lipid according to any one of claims 1 to 6, 8 to 10, and 12 to 22, wherein L₂ is C₁~C₃ hydrocarbylene.

24. The PEG lipid according to claim 23, wherein L₂ is C₁~C₃ alkylene, C₂~C₃ alkenylene or C₂~C₃ alkynylene.

25. The PEG lipid according to claim 24, wherein L₂ is straight C₁~C₃ alkylene, preferably C₁ alkylene or C₂ alkylene.

26. The PEG lipid according to any one of claims 1 to 6, 8 to 10, and 12 to 26, wherein L₃ is C₁~C₂ hydrocarbylene.

27. The PEG lipid according to claim 26, wherein L₃ is C₁~C₂ alkylene, C₂ alkenylene or C₂ alkynylene.

28. The PEG lipid according to any one of claims 1 to 27, wherein n is any integer from 30 to 55.

29. The PEG lipid according to claim 28, wherein n is any integer from 35 to 50.

30. The PEG lipid according to claim 29, wherein n is any integer from 40 to 50.

31. The PEG lipid according to claim 30, wherein n is 45.

32. The PEG lipid according to claim 1, wherein the PEG lipid is: 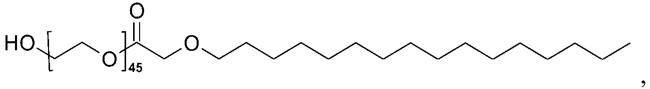 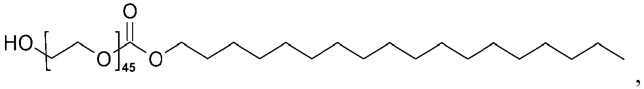 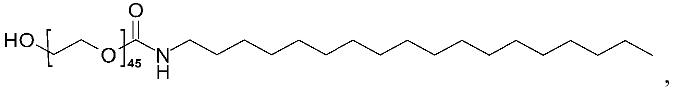 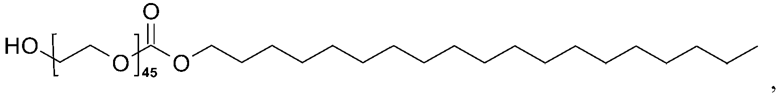 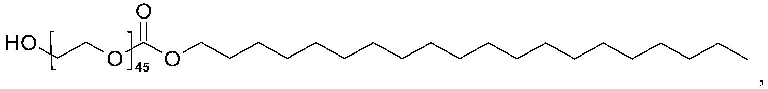 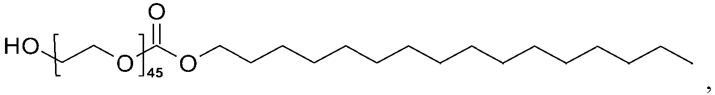 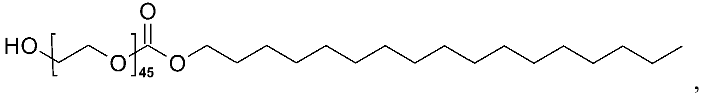 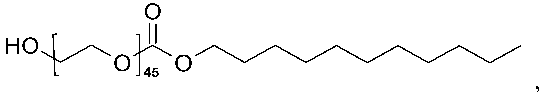 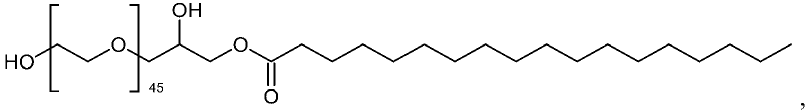 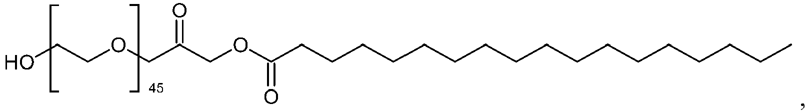 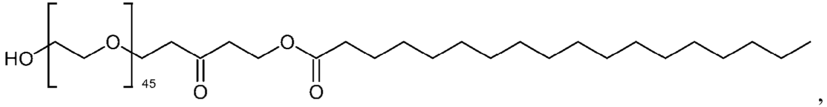 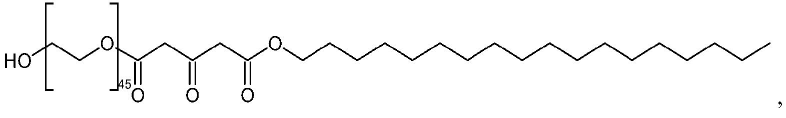 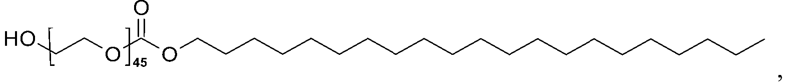 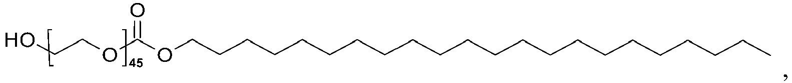 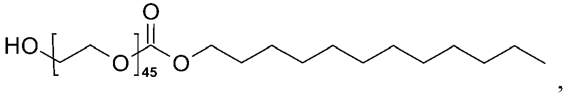 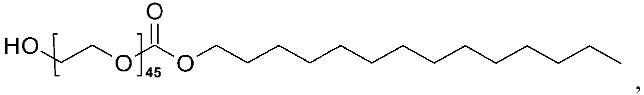 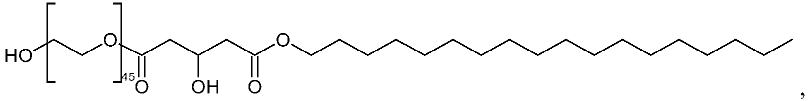 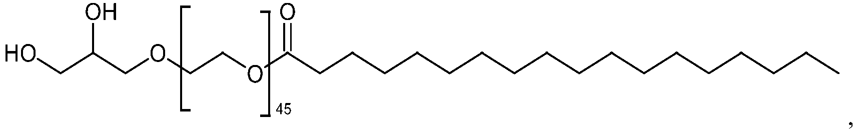 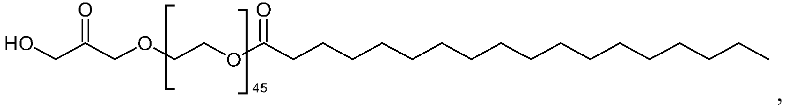 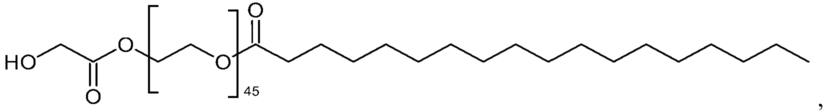 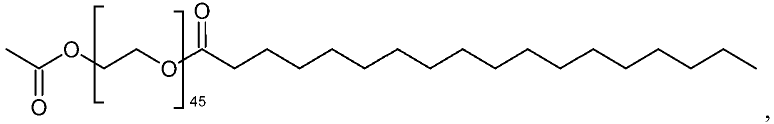 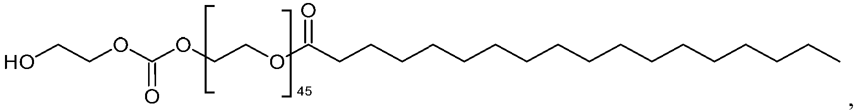 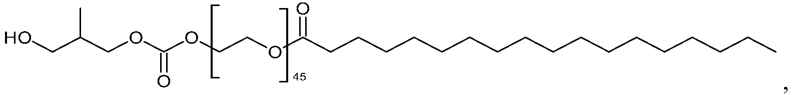 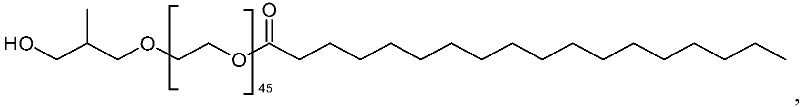 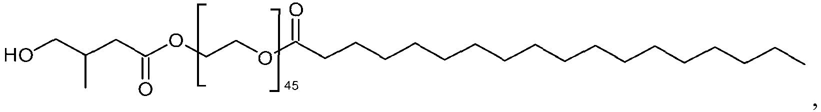 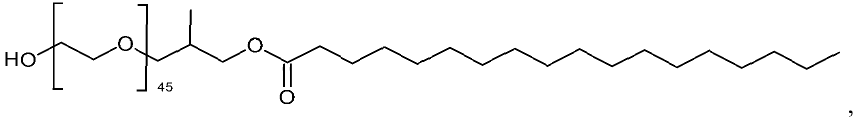 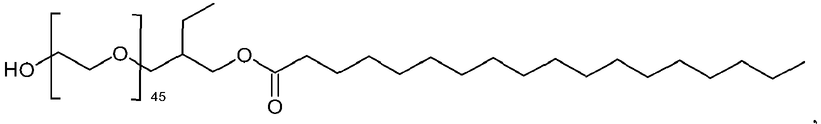 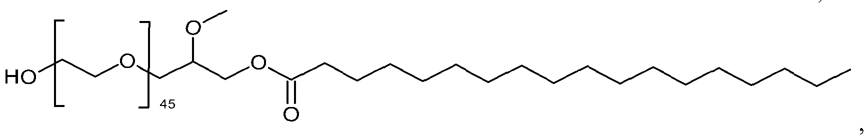 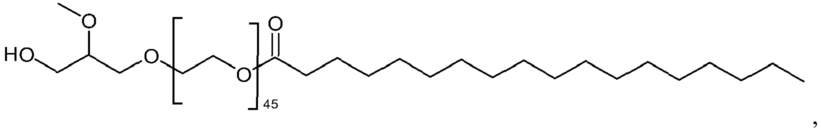 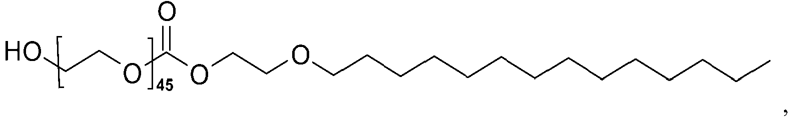 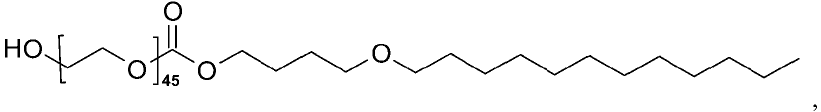 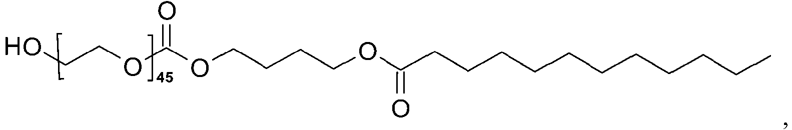 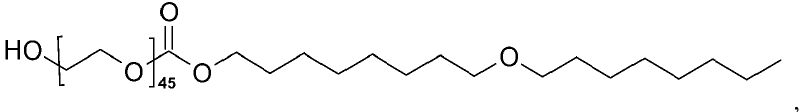 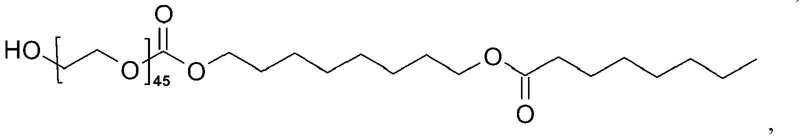 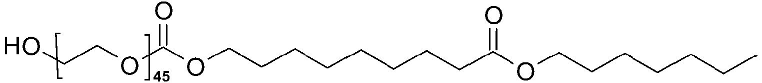 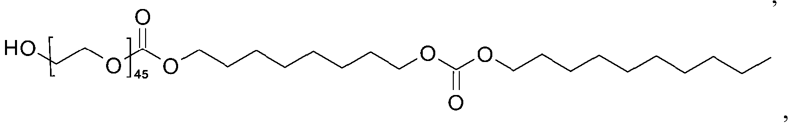 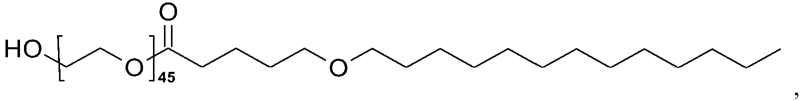 or

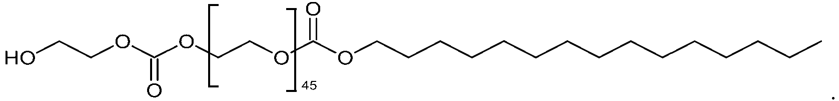

33. Use of the PEG lipid according to any one of claims 1 to 32 in the preparation of a lipid nanoparticle.

34. A lipid nanoparticle comprising the PEG lipid according to any one of claims 1 to 32.

35. The lipid nanoparticle according to claim 34, wherein the lipid nanoparticle further comprises an active ingredient;
wherein the active ingredient is preferably a pharmaceutically active ingredient, more preferably a nucleic acid;
preferably, the lipid nanoparticle further comprises one or more of a cationic lipid, a helper lipid, a structural lipid, and a polymeric lipid.

36. A pharmaceutical composition comprising the lipid nanoparticle according to claim 34 or 35, and a pharmaceutically acceptable carrier.

37. Use of the lipid nanoparticle according to claim 34 or 35 or the pharmaceutical composition according to claim 36 in the manufacture of a medicament;
preferably, the medicament is for use in gene therapy, genetic vaccination, protein replacement therapy, antisense therapy, or therapy via interfering RNA;
preferably, the lipid particle is used as a vehicle for transferring or delivering a pharmaceutically active ingredient; more preferably, the pharmaceutically active ingredient is a nucleic acid;
preferably, the medicament is for use in the treatment and/or prevention of a disease;
preferably, the medicament is for use in the treatment and/or prevention of one or more of rare diseases, cancers, infectious diseases, autoimmune diseases, metabolic diseases, neurological diseases, cardiovascular diseases, transplant rejection, inflammatory reactions, genetic diseases, and musculoskeletal diseases;
preferably, the medicament is a nucleic acid drug, wherein the nucleic acid includes at least one of RNA, messenger RNA (mRNA), antisense oligonucleotides, DNA, plasmids, ribosomal RNA (rRNA), microRNA (miRNA), transfer RNA (tRNA), small interfering RNA (siRNA), small nuclear RNA (snRNA), small hairpin RNA (shRNA), single-guide RNA (sgRNA) and Cas9 mRNA.

38. Use of the lipid nanoparticle according to claim 34 or 35 in the preparation of a vehicle for transferring or delivering an active ingredient;
preferably, the active ingredient is a pharmaceutically active ingredient;
preferably, the pharmaceutically active ingredient is a nucleic acid;
preferably, the nucleic acid includes at least one of RNA, messenger RNA (mRNA), antisense oligonucleotides, DNA, plasmids, ribosomal RNA (rRNA), microRNA (miRNA), transfer RNA (tRNA), small interfering RNA (siRNA), small nuclear RNA (snRNA), small hairpin RNA (shRNA), single-guide RNA (sgRNA) and Cas9 mRNA.
